# EUROPEAN PATENT APPLICATION

(11) **EP 1 332 763 A1**
(43) Date of publication of application: **06.08.2003**
(21) Application number: 01981043.1
(22) Date of filing: 09.11.2001
(51) Int. Cl.: A61K 45/00, A61P 3/10, C07D 267/14

(54) **HIGH-DENSITY LIPOPROTEIN-CHOLESTEROL LEVEL ELEVATING AGENT**

(30) Priority: 09.11.2000 JP 2000342607
(71) Applicant: Takeda Chemical Industries, Ltd., Osaka-shi, Osaka 541-0045 (JP)
(72) Inventor: NISHIMOTO, Tomoyuki, Suita-shi, Osaka (JP); TOZAWA, Ryuichi, Toyonaka-shi, Osaka 560-0021 (JP); KORI, Masakuni, Kobe-shi, Hyogo 651-2274 (JP); AMANO, Yuichiro, Osaka-shi, Osaka 533-0022 (JP)
(74) Representative: von Kreisler, Alek, Dipl.-Chem.
(86) International application number: JP0109802
(87) International publication number: WO02038180

(57) **Abstract**

A novel high-density lipoprotein(HDL)-cholesterol level elevating agent containing a compound which has a squalene synthase inhibitory activity.

## Description

### Technical Field

The present invention relates to a high density lipoprotein (HDL)-cholesterol level elevating agent comprising a compound having squalene synthase inhibitory activity. The present invention also relates to an agent for preventing or treating familial hypercholesterolemia comprising a compound having squalene synthase inhibitory activity.

### Background Art

Abnormal increase of the concentration of blood serum lipids is called hyperlipidemia or hyperlipemia. Blood serum lipids include such as cholesterol (cholesterol ester, free cholesterol), phospholipid (lecithin, sphingomyelin and the like), triglyceride (neutral lipid), free fatty acid, other sterols and the like, and specifically, the increase of cholesterol and triglyceride is a clinical problem (COMMON DISEASE SERIES No.19 hyperlipemia, Haruo Nakamura ed., published on October 10, 1991, by Nankodo).

Many epidemiological studies have shown that hypercholesterolemia, hypertension and smoking comprise the three major risk factors for atherosclerotic diseases such as myocardial infarction, angina pectoris and cerebral infarction. Appropriate control of blood cholesterol level is therefore critical to the prevention or treatment of atherosclerotic diseases, such as ischemic heart diseases. Examples of a drug for lowering a cholesterol level in blood include drugs that trap bile acid and inhibit its absorption such as Cholestyramine and Colestipol (for example, disclosed in U.S.P. 4,027,009) and drugs that inhibit acyl coenzyme A cholesterol acyltransferase (ACAT) and suppress intestinal absorption of cholesterol, such as Melinamide (disclosed in British Patent No. 1123004). In addition, drugs that suppress cholesterol biosynthesis, such as Lovastatin (disclosed in U.S.P. 4,231,938), Simvastatin (disclosed in U.S.P. 4,231,938 and 4,444,784) and Pravastatin (disclosed in U.S.P. 4,346,227) which inhibit 3-hydroxy-3-methylglutaryl coenzyme A (HMG-CoA) reductase, have drawn attention recently. But these drugs were not satisfactory, because when HMG-COA reductase is inhibited, however, not only biosynthesis of cholesterol, but also biosynthesis of other components essential to living organism such as ubiquinone, dolichol and heme A, are inhibited, resulting in side effects of concern.

Ubiquinone, dolichol, heme A and the like are known to be biosynthesized from farnesyl pyrophosphate in the cholesterol biosynthesis pathway; to prevent side effects due to their deficiency, it is desirable that the enzymes after farnesyl pyrophosphate in the cholesterol biosynthesis pathway are inhibited. For example, 2,3-oxidosqualene cyclase (EC 5.4.99.7) is an enzyme that converts 2,3-oxidosqualene to lanosterol; inhibition of 2,3-oxidosqualene cyclase results in decrease of cholesterol synthesis by inhibiting production of lanosterol which is used for conversion to cholesterol, and eventually leads to lowering blood cholesterol level. Also, as a result of inhibition of 2,3-oxidosqualene cyclase, the accumulated 2,3-oxidosqualene, is metabolically converted to oxysterol via dioxide squalene, which is known as an HMG-CoA reductase repressor [F. R. Taylor et al., Journal of Biological Chemistry, 1986, vol. 261 (32), pp. 15039-15044]. 2,3-Oxidosqualene cyclase inhibitors are therefore capable of inhibiting biosynthesis of cholesterol potently by the synergetic effect of HMG-CoA reductase suppression at the transcription level, in addition to the inhibition of one enzyme after farnesyl pyrophosphate in the cholesterol biosynthesis pathway. As 2,3-oxidosqualene cyclase inhibitors, diphenyl derivatives (disclosed in EP 464465), aminoalkoxybenzene derivatives (disclosed in EP 410359), piperidine derivatives (described by D. S. Dodd et al. in the Journal of Organic Chemistry, 1992, vol. 57, pp. 2794-2803 and 7226-7234; JP-A H4-234362), decalin derivatives, azadecalin derivatives and indane derivatives [described in WO 80/08450; Journal of Biological Chemistry, 1981, vol. 254, pp. 11258-11263; N. Gerst et al., Biochemical Pharmacology, 1988, vol. 37, pp. 1955-1964; M. W. Wannamaker, Journal of Medicinal Chemistry, 1992, vol. 35, pp. 3581-3583; JP-A H5-140112 and JP-A S64-3144], 2-aza-2,3-dihydrosqualene and 2,3-epiminosqualene [described by A. Duriatti et al. in Biochemical Pharmacology, 1985, vol. 34, pp. 2765-2777], squalenoid epoxide vinyl ether [described by M. Ceruti et al. in the Journal of the Chemical Society, Perkin Transaction 1, 1988, pp. 461-469], 29-methyliden-2,3-oxidosqualene [described in the Journal of American Chemical Society, 1991, vol. 113, pp. 9673-9674], azacycloalkane derivatives (disclosed in JP-A H6-192256) and arylcycloalkylamine derivatives (JP-A H6-211763) and the like are reported.

Compounds that inhibit cholesterol biosynthesis are useful in the treatment of some syndromes, especially hypercholesterolemia and hyperlipidemia, which comprise risk factors for onset of atherosclerotic vascular lesions and diseases subsequent thereto, such as coronary artery disease (CHD), cerebral ischemia, claudicatio intermittents and necrosis. Major symptoms of atherosclerosis are lesionin blood vessel such as intimal proliferation of macrophages and vascular smooth muscle cells and cholesterol accumulation in such cells. It is a well-known that hypercholesterolemia, especially increased low-density lipoprotein (LDL)-cholesterol, is an important risk factor in the development of atherosclerosis. Since epidemiological studies have revealed that the incidence of coronary heart disease can be decreased by lowering blood cholesterol level [see Lipidology 2(4), 234, 1991], blood lipid-lowering agents have been used to suppress atherosclerotic vascular lesions. Such use, however, is for prophylaxis, rather than treatment.

Hepatic low density lipoprotein (LDL) receptor plays a major role for the homeostasis of cholesterol. Specifically, familial hypercholesterolemia, which is a heredopathy based on the abnormality of an LDL receptor gene, is one of the most frequently recognized genetic disorders, and is a cause of LDL hypercholesterolemia, xanthoma and juvenile atherosclerosis. The cholesterol that circulates as a form of LDL is removed from blood plasma by a very specific LDL receptor, and taken into a cell by uptaking of the receptor-mediating cell. After uptaking of the cell, the particles of LDL are disintegrated by lysosome, thereby cholesterol is released to enhance the intracellular concentration of the free cholesterol. The increased free cholesterol concentration sends a signal to a hepatic cell to decrease the transcription rate of a key enzyme gene in the cholesterol biosynthesis pathway, whereby the synthesis of new cholesterol is decreased. Furthermore, LDL receptor mRNA and protein are downregulated by the increased cholesterol in the cell, whereby the ability of the liver to remove the increased LDL cholesterol from blood plasma is deteriorated. Therefore, the mechanism that independently upregulates the LDL receptor is expected to decrease the concentration of cholesterol in blood plasma significantly, and a drug that upregulates LDL receptor can be a novel blood lipid lowering agent.

Furthermore, as a triglyceride lowering agent, fibric acid compounds such as clofibrate (British Patent No. 860303), fenofibrate (German Patent No. 2250327) and the like are used as a medicament.

Moreover, it has been known epidemiologically that a high density lipoprotein (HDL)-cholesterol suppresses the incidence of cardiac diseases, and it has been recognized that the HDL removes cholesterol from a foam cell, as a reason therefor.

On the other hand, as compounds having inhibitory activity for biosynthesis of choresterol by inhibiting squalene synthase, the following compounds are known: squalestatins (e.g., U.S.P. 5506262, U.S.P. 5430055, U.S.P. 5409950, U.S.P. 5369125, JP-A H7-173166, JP-A H9-124655, JP-A H9-227566, Annual Review of Microbiology, Vol.49, pp. 607-639, 1995, Journal of Medicinal Chemistry, Vol.38, pp. 3502-3513, 1995, Journal of Medicinal Chemistry, Vol.39, pp. 207-216, 1996, Journal of Medicinal Chemistry, Vol.39, pp. 1413-1422, 1996 and the like), phosphoric acid compounds and carboxylic acid compounds of substrate analogs (e.g., U.S. Patent No. 5374628, U.S. Patent No. 5441946, U.S.P. 5428028, JP-A H7-041554, W09504025, Journal of Medicinal Chemistry, Vol.38, pp. 2596-2605, 1995, Arzniemittel-Forschung Drug Research, Vol.46, pp. 759-762, 1996, Journal of Medicinal Chemistry, Vol.31, pp. 1869-1871, 1988, Journal of Medicinal Chemistry, Vol.39, pp. 657-660, 1996, Journal of Medicinal Chemistry, Vol.39, pp. 661-664, 1996 and the like), carboxylic acid derivatives (e.g., WO9740006, WO9633159, WO9521834, WO9748701, EP 645377, EP 645378, EP 814080, EP 790235, JP-A H7-173120, JP-A H10-316634, JP-A H10-298134, JP-A H10-298177, JP-A H10-316617, JP-A H9-136880, W02000-00458, W098-29380, Bioorganic Medicinal Chemistry Letters, Vol.5, pp. 1989-1994, 1995, Bioorganic Medicinal Chemistry Letters, Vol.6, pp. 463-466, 1996, Journal of Medicinal Chemistry, Vol.40, pp. 2123-2125, 1997 and the like), amine compounds such as quinuclidine derivatives and the like (e.g., U.S.P. 5385912, U.S.P. 5494918, U.S.P. 5395846, U.S.P. 5451596, JP-A H8-134067, JP-A 2000-169474, JP-A H10-152453, JP-A 2000-502716, WO9403541, WO9405660, WO9535295, WO9626938, WO9531458, WO9500146, WO9725043, WO9812170 and the like) and the like.

### OBJECTS OF THE INVENTION

There is no report that the compound having squalene synthase inhibitory activity is involved in the high density lipoprotein (HDL)-cholesterol content in blood, and that the compound specifically elevates the content in blood plasma. Furthermore, the development of a novel agent having sufficiently satisfying effect for prevention or treatment of hyperlipemia or prevention or treatment of familial hypercholesterolemia and the like has been desired. Therefore, the major object of the present invention is to provide a novel medicament having high density lipoprotein (HDL)-cholesterol level elevation action.

### Summary of the Invention

The present inventors have found firstly that a compound having squalene synthase inhibitory activity is useful as a high-density lipoprotein-cholesterol level elevating agent, and that the compound is clinically sufficiently useful for medicaments having prevention or treatment action for hyperlipemia, prevention or treatment action for familial hypercholesterolemia, and the like, which resulted in the completion of the present study.

Namely, the present invention relates to:
(1) a high-density lipoprotein-cholesterol level elevating agent comprising a compound having squalene synthase inhibitory activity or a salt thereof, or a prodrug thereof;
(2) the agent according to the above-mentioned (1), which is an agent for preventing or treating primary hypoalphalipoproteinemia;
(3) the agent according the above-mentioned (1), which is an agent for preventing or treating Tangier disease;
(4) the agent according the above-mentioned (1), wherein the compound having squalene synthase inhibitory activity is a compound represented by the formula: 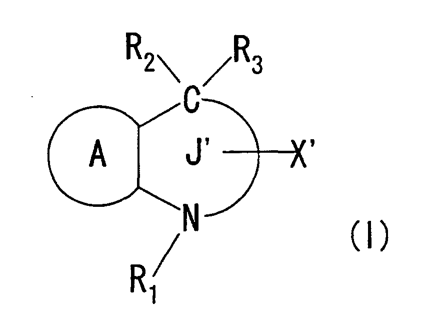 wherein R₁ is a hydrogen or an optionally substituted hydrocarbon group, R₂ and R₃ are the same or different and each is a hydrogen, an optionally substituted hydrocarbon group or an optionally substituted heterocyclic group, X' is a substituent constituted by an optionally esterified carboxyl group, an optionally substituted carbamoyl group, an optionally substituted hydroxy group, an optionally substituted amino group and an optionally substituted heterocyclic residue having a hydrogen atom that may be deprotonated, ring A is an optionally substituted benzene ring or an optionally substituted heterocyclic ring, ring J' is a 7- or 8-membered heterocyclic ring comprising three heteroatoms or less as the ring-constituting atoms, ring J' may have additional substituents besides R₁, R₂, R₃ and X',or a salt thereof;
(5) the agent according the above-mentioned (4), wherein R₁ is an optionally substituted aliphatic non-cyclic hydrocarbon group;
(6) the agent according to the above-mentioned (5), wherein the aliphatic non-cyclic hydrocarbon group is a branched alkyl group;
(7) the agent according to the above-mentioned (4), wherein R₂ or R₃ is an optionally substituted phenyl group;
(8) the agent according to the above-mentioned (4), wherein X' is an alkyl group substituted with an optionally esterified carboxyl group;
(9) the agent according to the above-mentioned (4), wherein X' is an alkyl group substituted with an optionally substituted heterocyclic residue having a hydrogen atom that may be deprotonated;
(10) The agent according to the above-mentioned (9), wherein the heterocyclic residue is 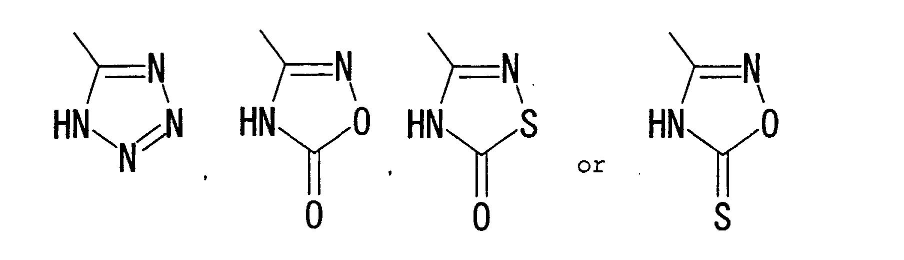
(11) the agent according to the above-mentioned (4), wherein X' is an alkyl group substituted with an optionally substituted carbamoyl group;
(12) the agent according to the above-mentioned (11), wherein the optionally substituted carbamoyl group is an optionally substituted cyclic aminocarbonyl group;
(13) the agent according to the above-mentioned (8), (9) or (11), wherein the alkyl group is a straight chain C₁₋₄ alkyl group;
(14) the agent according to the above-mentioned (4), wherein the heterocyclic ring represented by the ring A is 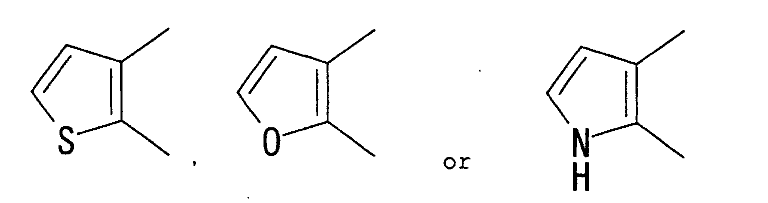
(15) the agent according to the above-mentioned (4), wherein the substituent of ring J' is an oxo or a thioxo;
(16) the agent according to the above-mentioned (4), wherein the fused ring consisting of the ring A and ring J' is 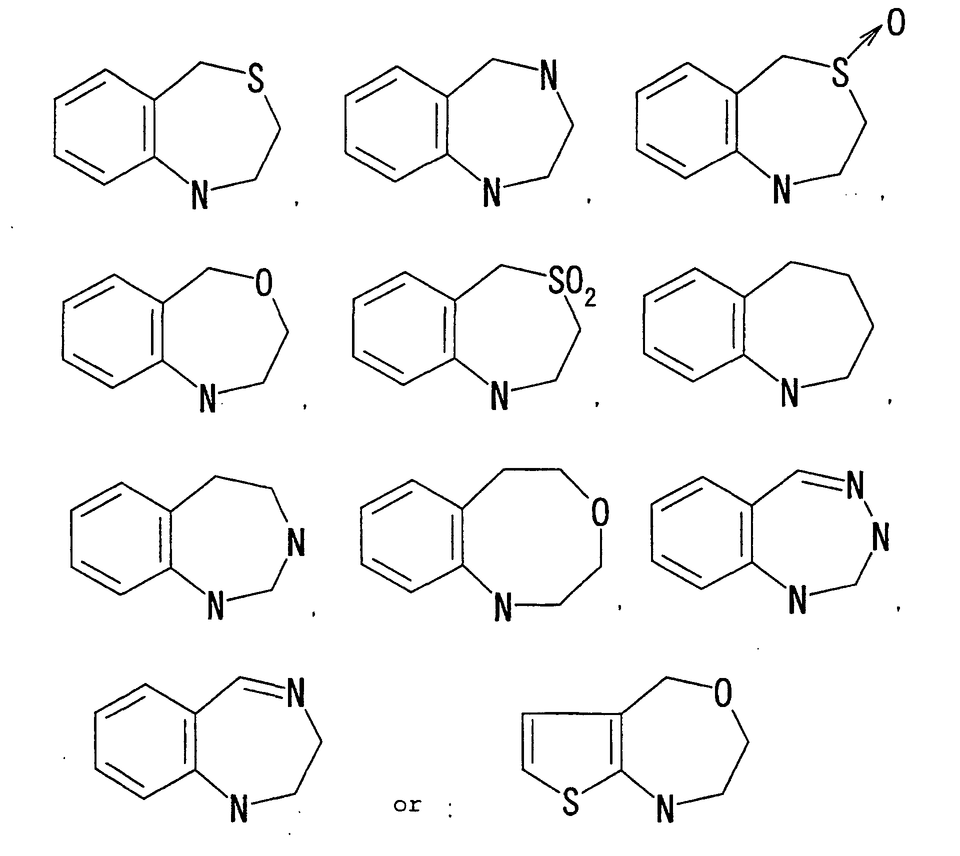
(17) the agent according to the above-mentioned (4), wherein R₂ and R₃ are each a hydrogen atom, an optionally substituted alkyl group, an optionally substituted phenyl group or an optionally substituted aromatic heterocyclic group;
(18) the agent according to the above-mentioned (1), comprising a compound represented by the formula: 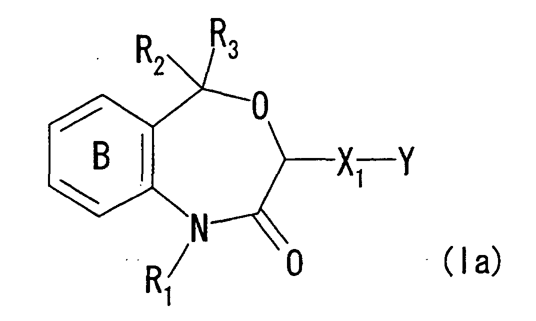 wherein R₁ is a hydrogen or an optionally substituted hydrocarbon group, R₂ and R₃ are the same or different and each is a hydrogen, an optionally substituted hydrocarbon group or an optionally substituted heterocyclic group, X₁ is a bond or divalent atomic chain, Y is an optionally esterified carboxyl group, an optionally substituted carbamoyl group, an optionally substituted hydroxy group, an optionally substituted amino group or an optionally substituted heterocyclic residue having a hydrogen atom which may be protonated and ring B is an optionally substituted benzene ring, or a salt thereof, or a prodrug thereof;
(19) the agent according to the above-mentioned (18), wherein R₁ is an optionally substituted aliphatic non-cyclic hydrocarbon group;
(20) the agent according to the above-mentioned (19), wherein the aliphatic non-cyclic hydrocarbon group is a branched alkyl group;
(21) the agent according to the above-mentioned (18), wherein R₂ or R₃ is an optionally substituted phenyl group;
(22) the agent according to the above-mentioned (18), wherein X₁ is a straight chain or a branched alkylene;
(23) the agent according to the above-mentioned (18), wherein X₁ is a straight chain C₁₋₄ alkylene;
(24) the agent according to the above-mentioned (18), wherein Y is an optionally esterified carboxyl group;
(25) the agent according to the above-mentioned (18), wherein Y is an optionally substituted carbamoyl group;
(26) the agent according to the above-mentioned (18), wherein Y is an optionally substituted heterocyclic residue having a hydrogen atom that may be deprotonated;
(27) the agent according to the above-mentioned (26), wherein the heterocyclic residue is 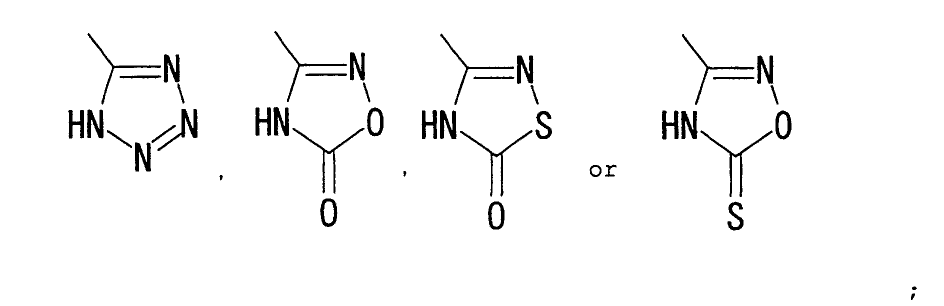
(28) the agent according to the above-mentioned (1), comprising a compound represented by the formula: 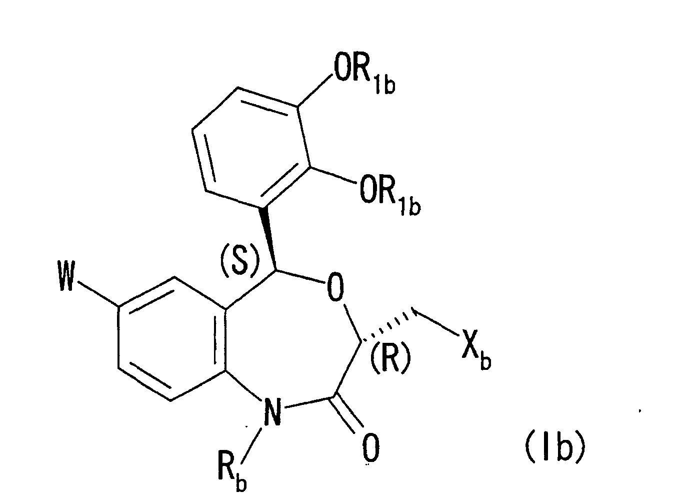 wherein R_{b} is a lower alkyl group optionally substituted with an optionally substituted hydroxy group, X_{b} is an optionally substituted carbamoyl group or an optionally substituted heterocyclic group having a hydrogen atom that may be deprotonated, R_{1b} is a lower alkyl group and W is a halogen atom or a salt thereof, or a prodrug thereof;
(29) the agent according the above-mentioned (28), wherein R_{b} is a C₁₋₆ alkyl optionally having 1 to 3 substituents selected from a hydroxy group, an acetyloxy, a propionyloxy, a t-butoxycarbonyloxy, a palmitoyloxy, a dimethylaminoacetyloxy and a 2-aminopropionyloxy;
(30) the agent according the above-mentioned (28), wherein R_{1b} is a methyl;
(31) the agent according the above-mentioned (28), wherein W is a chlorine atom;
(32) the agent according the above-mentioned (28), wherein X_{b} is a group represented by the formula: 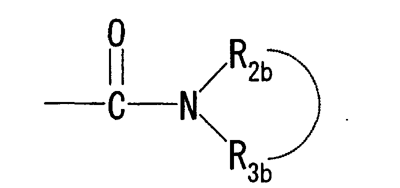 wherein R_{2b} and R_{3b} are each a hydrogen atom, an optionally substituted hydrocarbon group, an optionally substituted heterocyclic group or an acyl group, or R_{2b} and R_{3b} may form, together with the adjacent nitrogen atom, an optionally substituted 5- or 6-membered nitrogen-containing heterocyclic ring which may contain 1 to 3 heteroatoms selected from a nitrogen atom, a sulfur atom and an oxygen atom as the ring-constituting atoms;
(33) the agent according the above-mentioned (28), wherein X_{b} is a group represented by the formula: 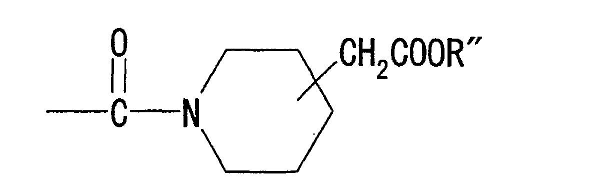 wherein R" is a hydrogen atom or a C₁₋₄ alkyl;
(34) the agent according to the above-mentioned (1), comprising a compound represented by the formula: 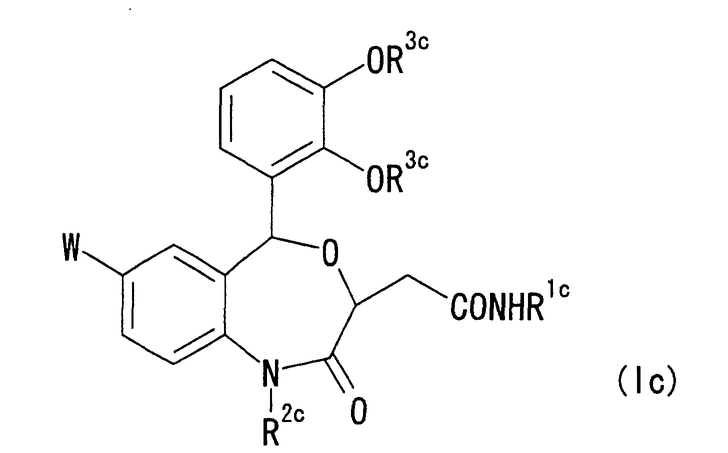 wherein R^{1c} is a 1-carboxyethyl group which may have substituents, a carboxy-C₃₋₆ straight chain alkyl group which may have substituent, a C₃₋₆ straight chain alkyl-sulfonyl group which may have substituent, a (carboxy-C₅₋₇ cycloalkyl)-C₁₋₃ alkyl group which may have substituent, or a group represented by the formula: -X¹-X²-Ar-X³-X⁴-COOH (wherein X¹ and X⁴ are each a bond or a C₁₋₄ alkylene group which may have substituent, X² and X³ are each a bond, -O-or -S- and Ar is a divalent aromatic ring group which may have substituent. Provided that when X¹ is a bond, X² is a bond, and when X⁴ is a bond, X³ is a bond), R^{2c} is a C₃₋₆ alkyl group optionally substituted with an alkanoyloxy group and/or a hydroxy group, R^{3c} is a lower alkyl group and W is a halogen atom or a salt thereof, or a prodrug thereof;
(35) the agent according the above-mentioned (34), wherein R^{1c} is a 3-carboxypropyl group; a 1-carboxyethyl group; or a C₃₋₆ straight chain alkyl-sulfonyl group, a (carboxy-C₅₋₇ cycloalkyl)-C₁₋₃ alkyl group, a (carboxyfuryl)-alkyl group, a carboxy-C₆₋₁₀ aryl group, a (carboxy-C₂₋₃ alkyl)-C₆₋₁₀ aryl group or a (carboxy-C₁₋₃ alkyl)-C₇₋₁₄ aralkyl group, each of which optionally has substituent;
(36) the agent according the above-mentioned (34), wherein R^{1c} is a (carboxy-C₁₋₄ alkyl)-C₆₋₁₀ aryl group which may have substituent;
(37) the agent according the above-mentioned (34), wherein R^{2c} is a C₃₋₆ alkyl group having an alkanoyloxy group and/or a hydroxy group;
(38) the agent according the above-mentioned (34), wherein R^{2c} is a C₃₋₆ alkyl group which may have 1 to 3 substituents selected from a hydroxy group, an acetoxy, a propionyloxy, a t-butoxycarbonyloxy and a palmitoyloxy;
(39) the agent according the above-mentioned (34), wherein R^{3c} is methyl group;
(40) the agent according to the above-mentioned (34), wherein W is chlorine atom;
(41) the agent according to the above-mentioned (34), wherein the 3-position has R-configuration and the 5-position has S-configuration;
(42) the agent according to the above-mentioned (1), comprising N-propanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, (2R)-2-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminopropionic acid, 3-[3-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminophenyl]propionic acid, 4-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminobutanoic acid, trans-4-([(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl-1-cyclohexanecarboxylic acid, trans-4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl-1-cyclohexanecarboxylic acid, 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-fluorophenyl]propionic acid, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methylphenyl]propionic acid, 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-methylphenyl]propionic acid, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl]phenyl]propionic acid, 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl]phenyl]propionic acid, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]propionic acid, 2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazen-3-yl]acetyl]amino]-4-fluorophenyl]propionic acid, 3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid, N-methanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide, N-[2-(pyrrolidin-1-yl)ethyl]-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide, N-[2-(pyrrolidin-1-yl)ethyl]-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-1-[3-acetoxy-2,2-dimethylpropyl)-7-chloro-5-(2,3-dimethoxyphenyl)-2-oxo-1,2,3,5-tetrahydro-4,1-benzoxazepin-3-yl]acetamide, N-methanesulfonyl-[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetamide, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid, N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester, N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester, (3R,5S)-7-chloro-5-(2,3-dimethoxyphenyl)-1-(3-hydroxy-2,2-dimethylpropyl)-1,2,3,5-tetrahydro-3-[1H (or 3H)-tetrazol-5-yl]methyl-4,1-benzoxazepin-2-one, (3R,5S)-7-chloro-5-(2,3-dimethoxyphenyl)-1-(3-hydroxy-2-hydroxymethyl-2-methylpropyl)-1,2,3,5-tetrahydro-3-[1H (or 3H)-tetrazol-5-yl]methyl-4,1-benzoxazepin-2-one, (3R,5S)-1-(3-acetoxy-2,2-dimethylpropyl-7-chloro-5-(2,3-dimethoxyphenyl)-1,2,3,5-tetrahydro-3-[1H (or 3H)-tetrazol-5-yl]methyl-4,1-benzoxazepin-2-one, (3R,5S)-1-(3-acetoxy-2-acetoxymethyl-2-methylpropyl)-7-chloro-5-(2,3-dimethoxyphenyl)-1,2,3,5-tetrahydro-3-[1H (or 3H)-tetrazol-5-yl]methyl-4,1-benzoxazepin-2-one, N-[2-(pyrrolidin-1-yl)ethyl]-[(3R,5S)-7-chloro-5-(2,3-dimethoxyphenyl)-1-neopentyl-2-oxo-1,2,3,5-tetrahydro-4,1-benzoxazepin-3-yl]acetamide, (3R,5S)-7-chloro-5-(2,3-dimethoxyphenyl)-1-neopentyl-2-oxo-1,2,3,5-tetrahydro-4,1-benzoxazepine-3-acetic acid, (3R,5S)-7-chloro-5-(2,4-dimethoxyphenyl)-1-neopentyl-2-oxo-1,2,3,5-tetrahydro-4,1-benzoxazepine-3-acetic acid, (3R,5S)-7-chloro-5-(4-ethoxy-2-methoxyphenyl)-1-neopentyl-2-oxo-1,2,3,5-tetrahydro-4,1-benzoxazepine-3-acetic acid, 4-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]butanoic acid, 5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]pentanoic acid, 5-[3-[[((3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]pentanoic acid,
   or a pharmaceutically acceptable salt thereof, or a prodrug thereof;
(43) an agent for preventing or treating familial hypercholesterolemia comprising a compound having squalene synthase inhibitory activity or a salt thereof, or a prodrug thereof;
(44) a method for elevating high density lipoprotein-cholesterol in a mammal, comprising administering a compound having squalene synthase inhibitory activity or a salt thereof, or a prodrug thereof to said mammal in an effective amount;
(45) a method for preventing or treating primary hypoalphalipoproteinemia in a mammal, comprising administering a compound having squalene synthase inhibitory activity or a salt thereof, or a prodrug thereof to said mammal in an effective amount;
(46) a method for preventing or treating Tangier disease in a mammal, comprising administering a compound having squalene synthase inhibitory activity or a salt thereof, or a prodrug thereof to said mammal in an effective amount;
(47) a method for preventing or treating familial hypercholesterolemia in a mammal, comprising administering a compound having squalene synthase inhibitory activity or a salt thereof, or a prodrug thereof to said mammal in an effective amount;
(48) a method for preventing or treating hyperlipemia or arterial sclerosis, comprising elevating high density lipoprotein-cholesterol in a mammal by administering a compound having squalene synthase inhibitory activity or a salt thereof, or a prodrug thereof to said mammal in an effective amount;
(49) use of a compound having squalene synthase inhibitory activity or a salt thereof, or a prodrug thereof, for the production of a medicament for elevating high density lipoprotein-cholesterol;
(50) use of a compound having squalene synthase inhibitory activity or a salt thereof, or a prodrug thereof, for the production of a medicament for preventing or treating primary hypoalphalipoproteinemia;
(51) use of a compound having squalene synthase inhibitory activity or a salt thereof, or a prodrug thereof, for the production of a medicament for preventing or treating Tangier disease;
(52) use of a compound having squalene synthase inhibitory activity or a salt thereof, or a prodrug thereof, for the production of a medicament for preventing or treating familial hypercholesterolemia; and the like.

### Detailed Description of the Invention

The "compound having squalene synthase inhibitory activity" used in the present invention may be any compound so long it posseses squalene synthase inhibitory activity, and includes compounds disclosed in the following documents, such as squalestatines (e.g., U.S.P. 5506262, U.S.P. 5430055, U.S.P. 5409950, U.S.P. 5369125, JP-A H7-173166, JP-A 124655/1997, Japanese Patent Publication No. H9-227566, Annual Review of Microbiology, Vol.49, 607-639, 1995, Journal of Medicinal Chemistry, Vol.38, 3502-3513, 1995, Journal of Medicinal Chemistry, Vol.39, 207-216, 1996, Journal of Medicinal Chemistry, Vol.39, 1413-1422, 1996 and the like), phosphoric acid compounds and carboxylic acid compounds of substrate analogs (e.g., U.S.P. 5374628, U.S.P. 5441946, U.S.P. 5428028, JP-A H7-041554, W09504025, Journal of Medicinal Chemistry, Vol.38, 2596-2605, 1995, Arzniemittel-Forschung Drug Research, Vol.46, 759-762, 1996, Journal of Medicinal Chemistry, Vol.31, 1869-1871, 1988, Journal of Medicinal Chemistry, Vol.39, 657-660, 1996, Journal of Medicinal Chemistry, Vol.39, 661-664, 1996 and the like), carboxylic acid derivatives (e.g., W09740006, WO9633159, WO9521834, WO9748701, EP 645377, EP 645378, EP 814080, EP 790235, JP-A H7-173120, JP-A H10-316634, JP-A H10-298134, JP-A H10-298177, JP-A H10-316617, JP-A H9-136880, JP-A 2001-187777, W02000-00458, W098-29380, Bioorganic Medicinal Chemistry Letters, Vol.5, 1989-1994, 1995, Bioorganic Medicinal Chemistry Letters, Vol.6, pp. 463-466, 1996, Journal of Medicinal Chemistry, Vol.40, pp. 2123-2125, 1997 and the like), amine compounds such as quinuclidine derivatives and the like (e.g., U.S.P. 5385912, U.S.P. 5494918, U.S.P. 5395846, U.S.P. 5451596, JP-A H8-134067, JP-A 2000-169474, JP-A H10-152453, JP-A 2000-502716, WO9403541, W09405660, W09535295, W09626938, W09531458, W09500146, W09725043, W09812170 and the like) and the like, specification of Japanese Patent Application No. 2000-190253 (PCT/JP01/05347), U.S.P. 6207644 and the like, and the like. Of these, a compound represented by the formula: 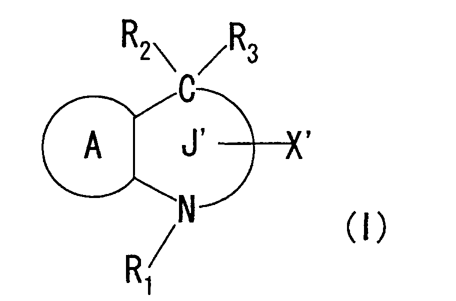 wherein R₁ is a hydrogen or an optionally substituted hydrocarbon group, R₂ and R₃ are the same or different and each is a hydrogen, an optionally substituted hydrocarbon group or an optionally substituted heterocyclic group, X' is a substituent selected from the group consisting of an optionally esterified carboxyl group, an optionally substituted carbamoyl group, an optionally substituted hydroxy group, an optionally substituted amino group or a substituent optionally substituted heterocyclic residue having a hydrogen atom that may be deprotonated, ring A is an optionally substituted benzene ring or an optionally substituted heterocyclic ring, ring J' is a 7- or 8-membered heterocyclic ring containing three heteroatoms or less as ring-constituting atom, and ring J' may have additional substituents besides R₁, R₂, R₃ and X'; or a compound represented by the formula: 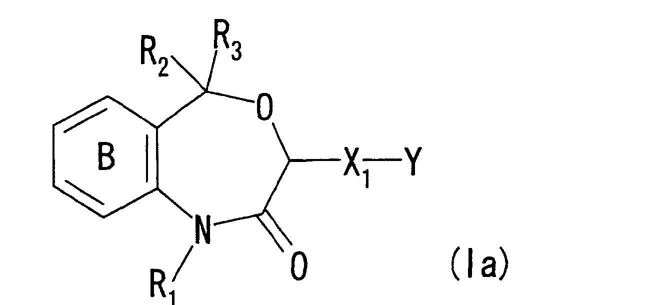 wherein R₁ is a hydrogen or an optionally substituted hydrocarbon group, R₂ and R₃ are the same or different and each is a hydrogen, an optionally substituted hydrocarbon group or an optionally substituted heterocyclic group, X₁ is a bond or divalent atomic chain, Y is an optionally esterified carboxyl group, an optionally substituted carbamoyl group, an optionally substituted hydroxy group, an optionally substituted amino group or an optionally substituted heterocyclic residue having a hydrogen atom which may be protonated, ring B is an optionally substituted benzene ring, and the like is preferably used.

The other squalene synthase inhibitors include, A-104109 (Abbott Laboratories), F-10863-A (Sankyo Co., Ltd.), ER-28448, ER-27856 (ER-28448 prodrug) and quinuclidine derivatives (Eisai Co., Ltd.), RPR-107393 (Rhone Poulenc Rorer S.A.), thiadiazole derivatives (Novo Nordisk A/S), isopropylamine derivatives (Yamanouchi Pharmaceutical Co., Ltd.), isoquinuclidine derivatives (Kotobuki Pharmaceutical Co., Ltd.), malonic acid derivatives, dioxolane derivatives (Nippon Kayaku Co., Ltd.), propionyl derivatives (Daiichi Pharmaceutical Co., Ltd.) and the like, and these squalene synthase inhibitors may also be used as an agent for the present invention.

The "compound having squalene synthase inhibitory activity" used in the present invention may be used in the form of salt, prodrug and the like.

As a salt of "compound having squalene synthase inhibitory activity" of the present invention, a salt acceptable as a pharmaceutical agents or a physiologically acceptable acid addition salt is preferred. For such salt, for example, inorganic acid (e.g., hydrochloric acid, phosphoric acid, hydrobromic acid, sulfuric acid and the like) or organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid and the like) and the like are used. Furthermore, when the "compound having squalene synthase inhibitory activity" of the present invention has an acid group such as carboxylic acid and the like, the "compound having squalene synthase inhibitory activity" may form a salt with inorganic bases (e.g., alkaline metals or alkaline earth metals such as sodium, potassium, calcium, magnesium and the like, or ammonia and the like) or organic bases (e.g., a tri-C₁₋₃ alkylamine such as triethylamine and the like).

A prodrug of compound having squalene synthase inhibitory activity or a salt thereof [hereinafter sometimes referred to as an SSI compound] of the present invention is a compound that converts to an SSI compound by the reaction with enzyme, gastric acid and the like under the physiological conditions in the body, that is, a compound that converts to an SSI compound by enzymatic oxidation, reduction or hydrolysis, or by hydrolysis by gastric acid . A prodrug of an SSI compound includes a compound wherein an amino group in SSI compound is acylated, alkylated, phosphorylated (e.g., an SSI compound whose amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated and the like); a compound wherein a hydroxy group in SSI compound is acylated, alkylated, phosphorylated, borated (e.g., an SSI compound whose hydroxy group is acetylated, palmitoylated, propanoylated, pivaloylated, succinylated, fumarylated, alanylated, dimethylaminomethylcarbonylated and the like); acompound wherein a carboxyl group in SSI compound is esterified or amidated (e.g., an SSI compound whose carboxyl group is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, methylamidated, and the like); and the like. These compounds can be prepared from an SSI compound by a method known *per se.*

A prodrug of an SSI compound may be a compound that converts to an SSI compound under physiological conditions as described in Development of Pharmaceutical Products, vol. 7, Molecular Design, 163-198, Hirokawa Shoten (1990).

The SSI compound may be a hydrate compound.

When an optically active form of an SSI compound is required, it can be obtained using an optically active starting material, or by resolution of the racemic compound using a conventional method. Furthermore, the SSI compound sometimes has asymmetry carbons in the molecule, and when two kinds of stereoisomers, R-configuration and S-configuration, are exist, each or the mixture of them are also encompassed in the present invention.

In the formulas (I) and (Ia), the hydrocarbon group of the "optionally substituted hydrocarbon group" represented R₁ includes an aliphatic chain (non-cyclic) hydrocarbon group, an alicyclic hydrocarbon group and an aryl group and the like. Of these, an aliphatic chain hydrocarbon group is preferred.

The aliphatic chain hydrocarbon group of the hydrocarbon group include, for example, a straight or branched chain aliphatic hydrocarbon group such as an alkyl group, an alkenyl group, an alkynyl group and the like. Of these, a branched alkyl group is preferred. The alkyl includes, for example, a C₁₋₇ alkyl such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl and the like. Of these, a C₃₋₅ alkyl such as n-propyl, isopropyl, isobutyl, neopentyl and the like are preferred, and isobutyl, neopentyl and the like are specifically preferred. The alkenyl group includes, for example, a C₂₋₆ alkenyl such as vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like. Of these, vinyl, allyl, isopropenyl, 2-methylallyl, 2-methyl-1-propenyl, 2-methyl-2-propenyl, 3-methyl-2-butenyl and the like are specifically preferred. The alkynyl group includes, for example, a C₂₋₆ alkynyl such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. Of these, ethynyl, 1-propynyl, 2-propynyl and the like are specifically preferred.

The alicyclic hydrocarbon group of the hydrocarbon group includes a saturated or unsaturated alicyclic hydrocarbon group such as a cycloalkyl group, a cycloalkenyl group, a cycloalkadienyl group and the like. As the cycloalkyl group, a cycloalkyl group having 3 to 9 carbon atoms are preferred, which includes cyclopropyl, cyclobutyl, cyclopentyl, cyclohexyl, cycloheptyl, cyclooctyl, cyclononyl and the like, of which a C₃₋₆ cycloalkyl group such as cyclopropyl, cyclobutyl, cyclopentyl, cyclohexyl and the like are preferred. The cycloalkenyl group includes, for example, a C₅₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl and the like. The cycloalkadienyl group includes, for example, C₅₋₆ cycloalkadienyl group such as 2,4-cyclopentadien-1-yl, 2,4-cyclohexadien-1-yl, 2,5-cyclohexadien-1-yl and the like.

The aryl group of the hydrocarbon group includes a monocyclic or fused polycyclic aromatic hydrocarbon group having 6 to 16 carbon atoms, such as phenyl, naphthyl, anthryl, phenanthryl, acenaphthylenyl and the like. Of these, a C₆₋₁₀ aryl group such as phenyl, 1-naphthyl, 2-naphthyl and the like are specifically preferred.

The substituent of the "optionally substituted hydrocarbon group" represented by R₁ includes an optionally substituted aryl group, an optionally substituted cycloalkyl group, an optionally substituted cycloalkenyl group, an optionally substituted heterocyclic group, an optionally substituted amino group, an optionally substituted hydroxy group, an optionally substituted thiol group, a halogen (e.g., fluorine, chlorine, bromine, iodine), an oxo and the like, and the hydrocarbon group may be substituted with any 1 to 5 (preferably 1 to 3) of these substituents at the substitutable positions. The aryl group of the optionally substituted aryl group includes a C₆₋₁₆ aryl group such as phenyl, naphthyl, anthryl, phenanthryl, acenaphthylenyl and the like. Of these, a C₆₋ ₁₀ aryl group such as phenyl, 1-naphthyl, 2-naphthyl and the like are preferred. The substituents of the optionally substituted aryl include a C₁₋₃ alkoxy group (e.g., methoxy, ethoxy, propoxy and the like), a halogen atom (e.g., fluorine, chlorine, bromine, iodine), a C₁₋₃ alkyl group (e.g., methyl, ethyl, propyl and the like) and the like, and the aryl group may be substituted with one or two of these substituents. The cycloalkyl group of the optionally substituted cycloalkyl group includes a C₃₋₇ cycloalkyl group such as cyclopropyl, cyclobutyl, cyclopentyl, cyclohexyl, cycloheptyl and the like. The kind and number of the substituents of the optionally substituted cycloalkyl group include those similar to the above-exemplified substituent of the optionally substituted aryl group. The cycloalkenyl group of the optionally substituted cycloalkenyl group includes a C₃₋₆ cycloalkenyl group such as cyclopropenyl, cyclobutenyl, cyclopentenyl, cyclohexenyl and the like. The kind and number of the substituents of the optionally substituted cycloalkenyl group include those similar to the above-exemplified substituent of the optionally substituted aryl group. The heterocyclic group of the optionally substituted heterocyclic group include an aromatic heterocyclic group and a saturated or unsaturated non-aromatic heterocyclic group (an aliphatic heterocyclic group), each of which has at least one, preferably 1 to 4 heteroatoms selected from an oxygen, a sulfur and a nitrogen as the ring-constituting atoms (ring atoms), and an aromatic heterocyclic group is preferred. The aromatic heterocyclic group include a 5 to 6-membered aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like) and an aromatic fused heterocyclic group in which two or three 5- or 6-membered rings are fused (e.g.: benzofuranyl, isobenzofuranyl, benzo[b]thienyl, indolyl, isoindolyl, 1H-indazolyl, benzimidazolyl, benzoxazolyl, 1,2-benzoisoxazolyl, benzothiazolyl, 1,2-benzoisothiazolyl, 1H-benzotriazolyl, quinolyl, isoquinolyl, cynnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenathridinyl, phenathrolinyl, indolizinyl, pyrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like). Of these, a 5 to 6-membered aromatic monocyclic heterocyclic group such as furyl, thienyl, indolyl, isoindolyl, pyrazinyl, pyridyl, pyrimidinyl and the like are preferred. The non-aromatic heterocyclic group include for example a 4 to 8-membered non-aromatic heterocyclic group such as oxiranyl, azetidinyl, oxethanyl, thiethanyl, pyrrolidinyl, tetrahydrofuryl, thiolanyl, piperidyl, tetrahydropyranyl, morpholinyl, thiomorpholinyl, piperazinyl and the like. The optionally substituted heterocyclic group may have 1 to 4, preferably 1 or 2 substituents, and such substituents includes a C₁₋₃ alkyl group (e.g.: methyl, ethyl, propyl and the like) and the like. The substituents of the optionally substituted amino group (including an amino group, a mono-or di-substituted amino group), optionally substituted hydroxy group and optionally substituted thiol group include for example a lower (C₁₋₃) alkyl (e.g., methyl, ethyl, propyl and the like) and the like. Furthermore, when the hydrocarbon group of the optionally substituted hydrocarbon group represented by R₁ is an alicyclic hydrocarbon group or an aryl group, the substituents may include additionally C₁₋₃ alkyl group (e.g., methyl, ethyl, propyl and the like).

Moreover, as mentioned above, R₁ may have an oxo group as a substituent, and R₁ also includes a carboxylic acyl group, which is thus oxo-substituted hydrocarbon group. Such examples thereof include, for example, an optionally substituted C₁₋₆ acyl group (e.g., formyl, acetyl, propionyl, butyryl, isobutyryl, valeryl, isovaleryl, pivaloyl, hexanoyl, dimethylacetyl, trimethylacetyl and the like) and the like. The acyl group may also have 1 to 5 substituents at the substitutable positions, and such substituents include a halogen (e.g., fluorine, chlorine, bromine).

In the formula (I) and (Ia), the "optionally substituted hydrocarbon group" represented by R₂ and R₃ includes groups those exemplified as groups of the "optionally substituted hydrocarbon group" represented by R₁. Provided that the alkyl group, an aryl group and the substituents thereof may also include the following groups. Namely, the alkyl group of the "optionally substituted alkyl group" includes a lower C₁₋₆ alkyl group (e.g.: methyl, ethyl, n-propyl, isopropyl, butyl, isobutyl, sec-butyl, tert-butyl, pentyl, isopentyl, neopentyl, hexyl, isohexyl and the like), preferably a C₁₋₄ alkyl group such as methyl, ethyl, propyl, isopropyl, butyl, tert-butyl and the like. For example, such optionally substituted alkyl group may have 1 to 4 substituents, and such substituent includes a halogen (e.g., fluorine, chlorine, bromine, iodine), a lower C₁₋₄ alkoxy group (e.g., methoxy, ethoxy, propoxy, isopropoxy, butoxy, tert-butoxy and the like) and the like.

The "optionally substituted aryl group" includes a monocyclic or fused polycyclic aromatic hydrocarbon group such as phenyl, naphthyl, anthryl, phenanthryl, acenaphthylenyl and the like. Of these, phenyl is specifically preferred.

The substituents of the "optionally substituted aryl group" include a halogen (e.g., fluorine, chlorine, bromine, iodine and the like), an optionally substituted lower alkyl, an optionally substituted lower alkoxy, an optionally substituted hydroxy group, a nitro, a cyano and the like, and the aryl group may be substituted with the same or different 1 to 3 (preferably 1 or 2) of these substituents. The lower alkyl includes for example a C₁₋₄ alkyl group such as methyl, ethyl, n-propyl, isopropyl, n-butyl, isobutyl, sec-butyl, tert-butyl and the like, and methyl, ethyl are specifically preferred. The lower alkoxy includes a C₁₋₄ alkoxy group such as methoxy, ethoxy, n-propoxy, isopropoxy, n-butoxy, isobutoxy, sec-butoxy, tert-butoxy and the like, and methoxy, ethoxy are specifically preferred. The substituents of the optionally substituted lower alkyl group or the optionally substituted lower alkoxy group include a halogen atom (e.g., fluorine, chlorine, bromine, iodine and the like) and the like, and the alkyl or alkoxy may be substituted with 1 to 5 of these substituents. The substituents of the optionally substituted hydroxy group includes a lower (C₁₋₄) alkyl group (e.g., methyl, ethyl, propyl, isopropyl, butyl, t-butyl and the like), a C₃₋₆ cycloalkyl group (cyclopropyl, cyclobutyl, cyclopentyl, cyclohexyl and the like), a C₆₋₁₀ aryl group (e.g., phenyl, 1-naphthyl, 2-naphthyl and the like), a C₇₋₁₂ aralkyl group (e.g., benzyl, phenetyl and the like) and the like. Furthermore, the adjacent substituents of these substituents may form a ring together, and when the aryl group of the "optionally substituted aryl group" represented by R₂ or R₃ is a phenyl group, the groups represented by 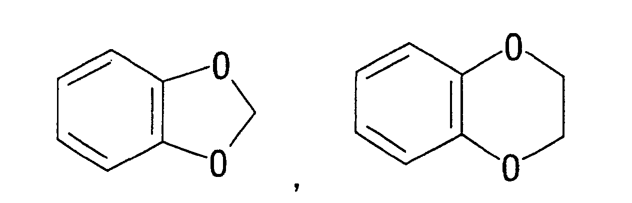 may be used. Such groups may be further substituted with 1 to 4 lower (C₁₋₃) alkyl groups (e.g., methyl, ethyl, propyl, isopropyl and the like) and the like.

The heterocyclic group of the "optionally substituted heterocyclic group" represented by R₂ and R₃ includes the heterocyclic groups described in detail in accordance with the substituents of the "optionally substituted heterocyclic group" represented by R₁. Of these, a 5 to 6-membered aromatic monocyclic heterocyclic ring such as furyl, thienyl, indolyl, isoindolyl, pyrazinyl, pyridyl, pyrimidyl, imidazolyl and the like are specifically preferred. The substituents of the heterocyclic group include a C₁₋₃ alkyl (e.g., methyl, ethyl, propyl and the like) and the like, and the heterocyclic group may have 1 to 4 of these substituents.

Of the above-mentioned groups, as R₂ and R₃, an optionally substituted phenyl group are preferred, a substituted phenyl group is more preferred, and a phenyl substituted with 1 to 3, preferably 1 to 2 groups selected from a halogen such as chlorine, bromine and the like, a lower (C₁₋₃) alkoxy and the like, is specifically preferred. Furthermore, either R₂ or R₃ is preferably a hydrogen.

In the formula (I), the "substituent constituted by an optionally esterified carboxyl group" represented by X' includes an optionally esterified carboxyl group and a substituent having an optionally esterified carboxyl group. The optionally esterified carboxyl group includes those exemplified as the optionally esterified carboxyl group defined by Y below.

The "substituent constituted by an optionally substituted carbamoyl group" represented by X' includes an optionally substituted carbamoyl group and a substituent having an optionally substituted carbamoyl group. The optionally substituted carbamoyl group includes those exemplified as the optionally substituted carbamoyl group defined by Y below.

The "substituent constituted by an optionally substituted hydroxy group" represented by X' includes an optionally substituted hydroxy group and a substituent having an optionally substituted hydroxy group. The optionally substituted hydroxy group includes those exemplified as the optionally substituted hydroxy group defined by Y below.

The "substituent constituted by an optionally substituted amino group" represented by X' includes an optionally substituted amino group and a substituent having an optionally substituted amino group. The optionally substituted amino group includes those exemplified as the optionally substituted amino group defined by Y below.

The "substituent constituted by an optionally substituted heterocyclic residue having a hydrogen atom that may be deprotonated" represented by X' includes an optionally substituted heterocyclic residue having a hydrogen atom that may be deprotonated (i.e., having an active proton), and a substituent having an optionally substituted heterocyclic residue having a hydrogen atom that may be deprotonated. The optionally substituted heterocyclic residue includes groups exemplified as the optionally substituted heterocyclic residue having a hydrogen atom that may be deprotonated, which is defined by Y below.

X' includes for example a group represented by the formula (a): 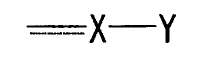 wherein X is a bond or a divalent or trivalent atomic chain , Y is an optionally esterified carboxyl group, an optionally substituted carbamoyl group, an optionally substituted hydroxy group, an optionally substituted amino group or an optionally substituted heterocyclic residue having a hydrogen atom that may be deprotonated, and the broken line portion is a single bond or a double bond.

In the formula (a), the "divalent atomic chain" represented by X may be a divalent chain in which the number of the atoms that constitutes the straight chain portion is preferably 1 to 7, more preferably 1 to 4, and the chain may have a side chain.

For example, the group represented by 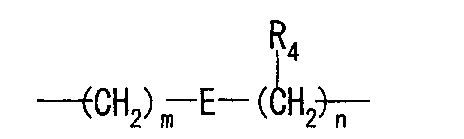 wherein each m and n is 0, 1, 2 or 3 independently, E is a bond or an oxygen atom, a sulfur atom, a sulfoxide, a sulfone, -N(R₅)-, -NHCO-, -CON(R₆)- or -NHCONH-, is exemplified. Here R₄ and R₆ is a hydrogen, an optionally substituted lower alkyl group, an optionally substituted aralkyl group or an optionally substituted phenyl group. R₅ is a hydrogen, lower alkyl group, aralkyl group or acyl group.

The alkyl group of the "optionally substituted lower alkyl group" represented by R₄ and R₆ includes a straight or branched lower C₁₋₆ alkyl group (e.g., methyl, ethyl, n-propyl, isopropyl, n-butyl, isobutyl, tert-butyl, n-pentyl, isopentyl, neopentyl and the like). The optionally substituted lower alkyl group may have 1 to 4, preferably 1 or 2 substituents, and these substituents include an aromatic heterocyclic group (e.g., a 5 to 6-membered aromatic heterocyclic ring containing 1 to 4 heteroatoms selected from N, O, S, such as furyl, thienyl, indolyl, isoindolyl, pyrazinyl, pyridyl, pyrimidyl, imidazolyl and the like), an optionally substituted amino group, an optionally substituted hydroxy group, an optionally substituted thiol group, an optionally esterified carboxyl group, a halogen atom (e.g., fluorine, chlorine, bromine, iodine) and the like. The substituents of the optionally substituted amino group (amino group or mono- or di-substituted amino group), optionally substituted hydroxy group and optionally substituted thiol group include a lower (C₁₋₃) alkyl (e.g., methyl, ethyl, propyl and the like) and the like. The optionally esterified carboxyl group includes a C₂₋₅ alkoxycarbonyl and a C₇₋₁₁ aryloxycarbonyl such as methoxycarbonyl, ethoxycarbonyl, propoxycarbonyl, phenoxycarbonyl, 1-naphthoxycarbonyl and the like, preferably methoxycarbonyl, ethoxycarbonyl, propoxycarbonyl.

The aralkyl group of the "optionally substituted aralkyl group" represented by R₄ and R₆ includes a C₇-C₁₅ aralkyl group such as benzyl, naphthylmethyl, phenylpropyl, phenylbutyl and the like. The optionally substituted aralkyl group may have 1 to 4, preferably 1 or 2 substituents, and such substituents include a halogen atom (e.g., fluorine, chlorine, bromine, iodine), a C₁₋₃ alkoxy group (e.g., methoxy, ethoxy, propoxy group), a hydroxy group, an amino group, a carboxyl group, a sulfhydryl group and the like.

The substituents of the "optionally substituted phenyl group" represented by R₄ and R₆ includes a halogen atom (e.g., fluorine, chlorine, bromine, iodine), a C₁₋₃ alkoxy (e.g., methoxy, ethoxy, propoxy and the like), a C₁₋₃ alkyl (e.g., methyl, ethyl, propyl) and the like.

R₄ may be different at each methylene chain.

The "lower alkyl group" and "aralkyl group" represented by R₅ each includes a lower C₁₋₄ alkyl group (e.g., methyl, ethyl, propyl, butyl, tert-butyl and the like), a C₇₋₁₅ aralkyl group (e.g., benzyl, phenetyl, phenylpropyl, phenylbutyl, naphthylmethyl and the like).

The "acyl group" represented by R₅ includes a lower (C₁₋₆) alkanoyl group (e.g., formyl, acetyl, propionyl, butyryl, isobutyryl, valeryl, isovaleryl, pivaloyl, hexanoyl and the like), a lower (C₃₋₇) alkenoyl group (e.g., acryloyl, methacryloyl, crotonoyl, isocrotonoyl and the like), a C₄₋₇ cycloalkanecarbonyl group (e.g., cyclopropanecarbonyl group, cyclobutanecarbonyl group, cyclopentanecarbonyl group, cyclohexanecarbonyl group and the like), a lower (C₁₋₄) alkanesulfonyl group (e.g., mesyl, ethanesulfonyl, propanesulfonyl and the like), a C₇₋₁₄ aroyl group (e.g., benzoyl, p-toluoyl, 1-naphthoyl, 2-naphthoyl and the like), a C₆₋₁₀ aryl lower (C₂₋₄) alkanoyl group (e.g., phenylacetyl, phenylpropionyl, hydroatropoyl, phenylbutyryl and the like), a C₆₋₁₀ aryl lower (C₃₋₅) alkenoyl group (e.g., cynnamoyl, atropoyl and the like), a C₆₋₁₀ arenesulfonyl group (e.g., benzenesulfonyl, p-toluenesulfonyl group and the like) and the like.

Furthermore, X may be a carbon chain containing double bond or -L-CH(OH)- (L is a bond or a straight or branched alkylene chain). The "carbon chain containing double bond" includes a group containing preferably 1 to 7, more preferably 1 to 4 carbons that constitute the straight chain portion, and the chain may have a side chain. The double bond in the carbon chain is included in either or both of the straight chain portion and branched chain portion, and preferably included in the straight chain portion. While the number of the double bond included in the carbon chain is not specifically limited as possible, 1 or 2 is preferred.

The carbon chain containing double bond includes methyne, vinylene, propenylene, butenylene, butadienylene, methylpropenylene, ethylpropenylene, propylpropenylene, methylbutenylene, ethylbutenylene, propylbutenylene, methylbutadienylene, ethylbutadienylene, propylbutadienylene, pentenylene, hexenylene, heptenylene, pentadienylene, hexadienylene, heptadienylene and the like, preferably methyne, vinylene, propenylene, butenylene and butadienylene. When the carbon chain is trivalent, the carbon chain is linked to a substitutable carbon atom on the ring of the ring J' with a double bond.

The "straight or branched alkylene chain" represented by L includes a straight or branched alkylene chain having 1 to 6 carbon atoms, for example, a divalent group such as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene and the like, preferably, a divalent group having 1 to 3 carbon atoms such as methylene, ethylene, trimethylene, propylene and the like.

Of the above-mentioned groups, as X', a group represented by the formula (b):

―X₁―Y

wherein X₁ is a bond or a divalent atomic chain, Y is an optionally esterified carboxyl group, an optionally substituted carbamoyl group, an optionally substituted hydroxy group, an optionally substituted amino group or an optionally substituted heterocyclic residue having a hydrogen atom that may be deprotonated, is preferred.

In the formula (b), the divalent atomic chain represented by X₁ includes groups exemplified as the divalent atomic chain defined by the above-mentioned X.

In the formula (a) and (b), "divalent atomic chain" represented by X or X₁ preferably includes a straight or branched alkylene chain in which the number of the carbon atoms constituting the straight chain portion is 1 to 7 (more preferably 1 to 4). The alkylene chain includes a divalent group such as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene and the like, preferably a divalent group having 1 to 4 carbon atoms such as methylene, ethylene, trimethylene, propylene and the like.

In the formula (a) and (b), the "optionally esterified carboxyl group" represented by Y includes a lower C₂₋₇ alkoxycarbonyl (e.g., methoxycarbonyl, ethoxycarbonyl, propoxycarbonyl, isopropoxycarbonyl, butoxycarbonyl, tert-butoxycarbonyl, sec-butoxycarbonyl, pentyloxycarbonyl, isopentyloxycarbonyl, neopentyloxycarbonyl and the like), a C₇₋₁₄ aryloxycarbonyl (e.g., phenoxycarbonyl, 1-naphthoxycarbonyl), a C₈₋₁₂ aralkyloxycarbonyl (e.g., benzyloxycarbonyl and the like) and the like. Of these, carboxyl group, methoxycarbonyl, ethoxycarbonyl are preferred.

The substituents of the "optionally substituted carbamoyl group" represented by Y include an optionally substituted lower (C₁₋₆) alkyl (e.g., methyl, ethyl, n-propyl, isopropyl, butyl, isobutyl, sec-butyl, tert-butyl, pentyl, isopentyl, neopentyl, hexyl, isohexyl and the like), an optionally substituted C₃₋₆ cycloalkyl (e.g., cyclopropyl, cyclobutyl, cyclopentyl, cyclohexyl and the like), an optionally substituted C₆₋₁₄ aryl group (e.g., phenyl, 1-naphthyl, 2-naphthyl and the like), an optionally substituted C₇₋₁₁ aralkyl group (e.g., benzyl, phenetyl and the like) and the like. The carbamoyl group may be substituted with, similarly or differently, one or two of these substituents. The substituents of the optionally substituted lower (C₁₋₆) alkyl and optionally substituted C₃₋ ₆ cycloalkyl include a carboxyl group optionally esterified with a lower (C₁₋₅) alkyl (e.g., methyl, ethyl, propyl, isopropyl, butyl, t-butyl, pentyl, isopentyl, neopentyl), a 5 to 6-membered aromatic heterocyclic group containing 1 to 4 heteroatoms (e.g., furyl, thienyl, indolyl, isoindolyl, pyrazinyl, pyridyl, pyrimidyl, imidazolyl and the like), an amino group, a hydroxy group, a phenyl group and the like, and the alkyl group or cycloalkyl group may be substituted with, similarly or differently, 1 to 3 of these substituents. The substituents of the optionally substituted aryl group and optionally substituted aralkyl group include a halogen atom (e.g., fluorine, chlorine, bromine, iodine), a carboxyl group optionally esterified with a lower (C₁₋₄) alkyl group (e.g., methyl, ethyl, propyl, isopropyl, butyl, t-butyl and the like) and the like. Furtheremore, in the optionally substituted carbamoyl group, the two substituents on a nitrogen atom may form, together with the nitrogen atom, a cyclic amino group, and the examples of such cyclic amino group include 1-azetidinyl, 1-pyrrolidinyl, piperidino, morpholino, 1-piperazinyl and the like. The cyclic amino group may have additional substituents.

The substituents of the "optionally substituted hydroxy group" represented by Y include for example a lower (C₁₋₄) alkyl (e.g., methyl, ethyl, propyl, isopropyl, butyl, t-butyl and the like), a C₃₋₆ cycloalkyl group (e.g., cyclopropyl, cyclobutyl, cyclopentyl, cyclohexyl and the like), an optionally substituted C₆₋₁₀ aryl group (e.g., phenyl, 1-naphthyl, 2-naphthyl and the like), an optionally substituted C₇₋₁₁ aralkyl group (e.g., benzyl, phenetyl and the like) and the like. The substituents of the optionally substituted aryl group and optionally substituted aralkyl group include a halogen atom (e.g., fluorine, chlorine, bromine, iodine), a carboxyl group optionally esterifed with a lower (C₁₋₄) alkyl group (e.g., methyl, ethyl, propyl, isopropyl, butyl, t-butyl and the like) and the like.

The "optionally substituted amino group" represented by Y includes mono-substituted and di-substituted amino groups, and the substituents thereof include for example a lower (C₁₋₄) alkyl (e.g., methyl, ethyl, propyl, isopropyl, butyl, tert-butyl and the like), a C₃₋₆ cycloalkyl group (e.g., cyclopropyl, cyclobutyl, cyclopentyl, cyclohexyl and the like), an optionally substituted C₆₋₁₀ aryl group (e.g., phenyl, 1-naphthyl, 2-naphthyl and the like), an optionally substituted C₇₋₁₁ aralkyl group (e.g., benzyl, phenetyl and the like) and the like. The substituents of the optionally substituted aryl group and optionally substituted aralkyl group include a halogen atom (e.g., fluorine, chlorine, bromine, iodine), a carboxyl group optionally esterified with a lower (C₁₋₄) alkyl group (e.g., methyl, ethyl, propyl, isopropyl, butyl, tert-butyl and the like) and the like, and the aryl or aralkyl group may have 1 to 4, preferably 1 to 2 of these substituents. Furthermore, the two substituents on the nitrogen atom may form, together with the nitrogen atom, a cyclic amino group, and such cyclic amino group includes 1-azetidinyl, 1-pyrrolidinyl, piperidino, morpholino, 1-piperazinyl and the like. Additionally, the cyclic amino group may have additional substituents.

The heterocyclic residue of the "optionally substituted heterocyclic residue having a hydrogen atom that may be deprotonated" includes a 5 to 7-membered (preferably 5-membered) monocyclic heterocyclic residue containing at least one of N, S and O (preferably, a nitrogen-containing heterocyclic residue), and the residue preferably has a hydrogen atom that can be eliminated to form a proton. For example, a tetrazol-5-yl or a group represented by 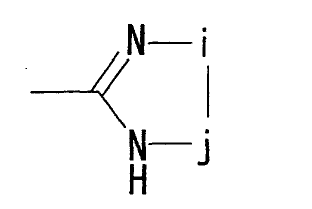 wherein i is -O- or -S-, j is >C=O, >C=S or >S(O)₂ (of these, 2,5-dihydro-5-oxo-1,2,4-oxadiazol-3-yl, 2,5-dihydro-5-thioxo-1,2,4-oxadiazol-3-yl, 2,5-dihydro-5-oxo-1,2,4-thiadiazol-3-yl are preferred) and the like are exemplified.

The above-mentioned heterocyclic residue may be protected with an optionally substituted lower alkyl group (preferably a C₁₋₄ alkyl) or an acyl group and the like. The optionally substituted lower alkyl group includes a C₁₋₄ alkyl optionally substituted with a phenyl which may be substituted with a C₁₋₃ alkyl, a nitro, or a C₁₋₃ alkoxy, or a C₁₋₃ alkoxy (methyl, triphenylmethyl, methoxymethyl, ethoxymethyl, p-methoxybenzyl, p-nitrobenzyl and the like) and the like. The acyl group includes a lower(C₂₋₅)alkanoyl, a benzoyl and the like.

Of the above-mentioned group, as X', an alkyl group substituted with an optionally esterified carboxyl group, an alkyl group substituted with an optionally substituted with a heterocyclic residue having a hydrogen atom that may be deprotonated, or an alkyl group substituted with an optionally substituted carbamoyl group, are preferred.

In the formula (I), the heterocyclic ring represented by ring A includes heterocyclic groups described in detail in accordance with the substituents of the hydrocarbon group represented by R₁, and of these, the groups represented by 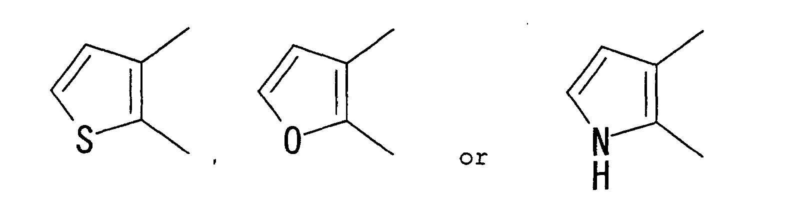 are preferred.

The substituents of the "optionally substituted benzene ring" and the "optionally substituted heterocyclic ring" represented by ring A include a halogen (e.g., fluorine, chlorine, bromine, iodine), an optionally substituted lower C₁₋₄ alkyl group (e.g., methyl, ethyl, propyl, butyl, tert-butyl and the like), an optionally substituted lower C₁₋₄ alkoxy group (e.g., methoxy, ethoxy, propoxy, isopropoxy, butoxy, tert-butoxy and the like), a hydroxy group, a nitro group, a cyano and the like. The ring A may have 1 to 3, preferably 1 to 2 of these substituents. Alternatively, the adjacent substituents of these substituents may form a ring. The substituents of the optionally substituted lower alkyl group or optionally substituted lower alkoxy group include a halogen atom (e.g., fluorine, chlorine, bromine, iodine) and the like, and 1 to 3 of these groups may be substituted at any positions. The ring A is preferably substituted with methoxy or chlorine atom, and specifically preferably substituted with chlorine atom.

In the formula (Ia), the substituents of the "optionally substituted benzene ring" represented by ring B include a halogen (e.g., fluorine, chlorine, bromine, iodine), an optionally substituted lower C₁₋₄ alkyl group (e.g., methyl, ethyl, propyl, butyl, tert-butyl and the like), an optionally substituted lower C₁₋₄ alkoxy group (e.g., methoxy, ethoxy, propoxy, isopropoxy, butoxy, tert-butoxy and the like), a hydroxy group, a nitro group, a cyano and the like. The ring B may have 1 to 3, preferably 1 or 2 of these substituents. Alternatively, the adjacent substituents of these substituents may form a ring together. The substituents of the optionally substituted lower alkyl group or optionally substituted lower alkoxy group include a halogen atom (e.g., fluorine, chlorine, bromine, iodine) and the like, and 1 to 3 of these groups may be substituted at any positions. The ring B is preferably substituted with a methoxy or a chlorine atom, and specifically preferably, substituted with a chlorine atom.

In the formula (I), the heterocyclic ring of the "7 to 8-membered heterocyclic ring having three heteroatoms or less as the ring-constituting atoms" represented by ring J' includes for example a 7 to 8-membered saturated or unsaturated heterocyclic ring containing at least one of O, S(O)_{q} (q is 0, 1 or 2) and N. Provided that the heteroatoms constituting the heterocyclic ring (ring-constituting atoms) is three or less.

Furthermore, the ring J' may have additional 1 or 2 substituents at the substitutable positions, besides the groups represented by R₁, R₂, R₃ and X'. The substituents include, when the substituents are bound to the nitrogen atom of the ring J', an alkyl group (e.g., a C₁₋₆ alkyl such as methyl, ethyl, n-propyl, isopropyl, n-butyl, isobutyl, tert-butyl, n-pentyl, isopentyl, neopentyl and the like), an acyl group (e.g., a C₁₋₄ acyl group such as formyl, acetyl, propionyl, butyroyl and the like) and the like. The alkyl group or acyl group may be further substituted with 1 to 5 halogen atoms (e.g., fluorine, chlorine, bromine, iodine). Alternatively, when the substituents are bound to the carbon atoms on the ring J', the substituents include an oxo, a thioxo, an optionally substituted hydroxy group, an optionally substituted amino group and the like. The optionally substituted hydroxy group and optionally substituted amino group include the groups exemplified as the "optionally substituted hydroxy group" and "optionally substituted amino group" defined by the above-mentioned Y.

As the ring J', preferred is those substituted with an oxo or a thioxo at the substitutable position in addition to the groups represented by R₁, R₂, R₃, and X'.

The fused ring consisting of the ring A and ring J' includes for example 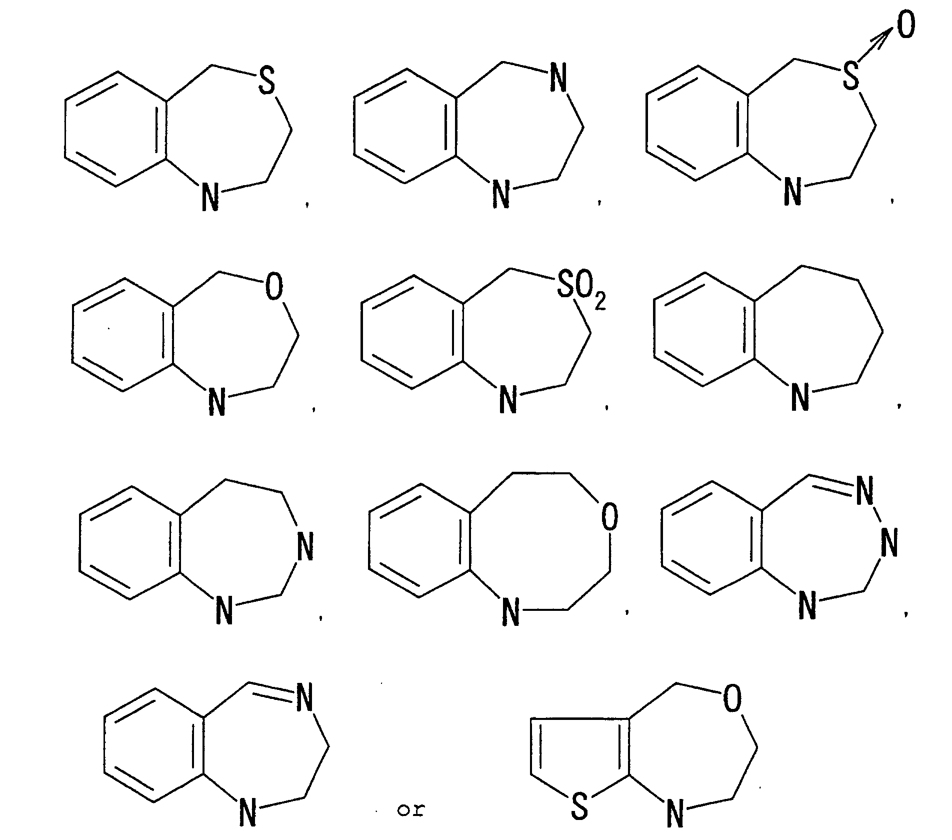 and the like.

As the formula (I), the formula represented by the formula (I') 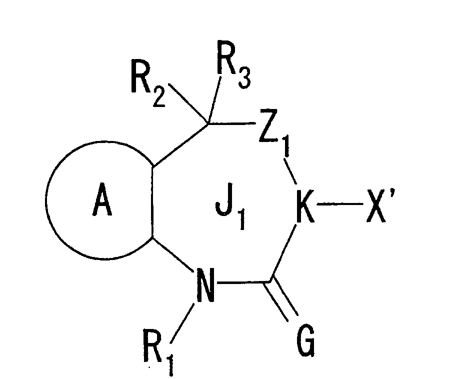 wherein R₁, R₂, R₃, X', ring A are as defined above. Ring J₁ is a 7-membered heterocyclic ring, Z₁ is -N(R₇)- (R₇ is a hydrogen, an alkyl group or an acyl group), -S(O)_{q}- (q is 0,1 or 2), -CH₂- or -O-, K is C or N and G is O or S, is preferred.

In the formula (Ia) above, the alkyl group represented by R₇ includes a straight or branched lower C₁₋₆ alkyl group (e.g., methyl, ethyl, n-propyl, isopropyl, n-butyl, isobutyl, tert-butyl, n-pentyl, isopentyl, neopentyl and the like), which may be substituted with 1 to 5 halogen atoms (e.g., fluorine, chlorine, bromine, iodine).

The acyl group represented by R₇ includes a C₁₋₄ acyl group (e.g., formyl, acetyl, propionyl, butyroyl and the like), which may be substituted with 1 to 5 halogen atoms (e.g., fluorine, chlorine, bromine, iodine).

In the formula (I'), as Z₁, S(O)_{q} (q is 0, 1 or 2) and O are preferred. Furthermore, K is preferably C, and G is preferably O.

As the formula (I'), the formula (I''): 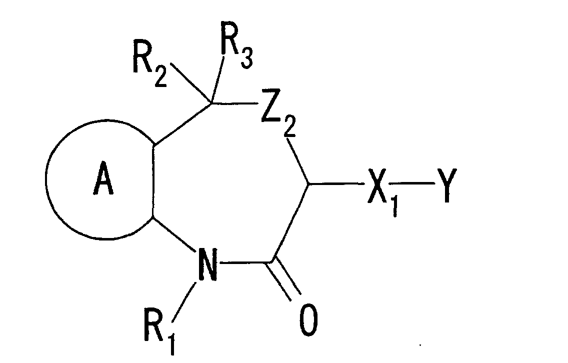 wherein R₁, R₂, R₃, X₁, Y, ring A are as defined above. Z₂ is S(O)_{q} (q is 0, 1 or 2) or O, is more preferred.

As the compound represented by the formula (I), a compound represented by the above-mentioned formula (Ia) 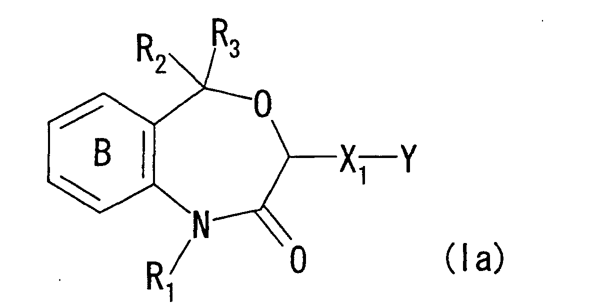 is preferred.

The formula (Ia) may be those represented by the formula (Ia'): 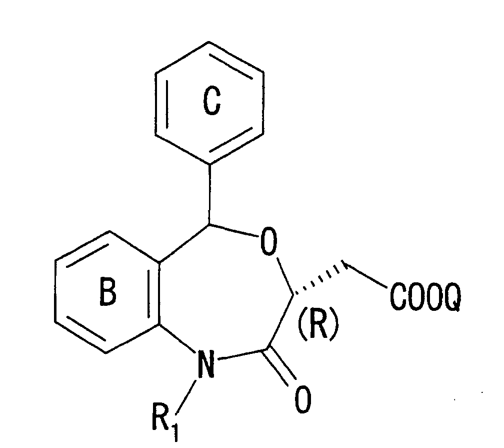 wherein R₁ and ring B are as defined above, Q is a hydrogen or a metal ion and ring C is an optionally substituted phenyl group. In the formula, the substituents on the 3-position and 5-position are directed to the opposite directions each other relative to the plane of the 7-membered ring, which represents trans, and (R) represents an R-configuration.

In the formula (Ia') above, the metal ion represented by Q includes sodium ion, potassium ion, calcium ion, alminum ion and the like, and of these, sodium ion, potassium ion is preferred.

The substituents of the "optionally substituted phenyl group" represented by the ring C include groups exemplified as the substituents of the "optionally substituted aryl group" explained as the examples of the "optionally substituted hydrocarbon group" defined by R₂ and R₃.

The salt of the compound represented by the formula (I) includes a pharmacologically acceptable salt thereof such as an inorganic salt such as hydrochloride, hydrobromide, sulfate, nitrate, phosphate and the like, an organic acid salt such as acetate, tartrate, citrate, fumarate, maleate, toluenesulfonate, methanesulfonate and the like, a metal salt such as sodium salt, potassium salt, calcium salt, alminum salt and the like, a base salt such as triethylamine salt, guanidine salt, ammonium salt, hydrazine salt, quinine salt, cinchonine salt and the like, and the like. Specifically, sodium salt is preferred.

The compounds of the formula (I) are specifically exemplified as follows: (3R,5S)-7-cyano-5-(2,3-dimethoxyphenyl)-1-neopentyl-2-oxo-1,2,3,5-tetrahydro-4,1-benzoxazepine-3-acetic acid, (3R,5S)-7-cyano-5-(2,4-dimethoxyphenyl)-1-neopentyl-2-oxo-1,2,3,5-tetrahydro-4,1-benzoxazepine-3-acetic acid, (3R,5S)-7-cyano-5-(2,3-methylenedioxyphenyl)-1-neopentyl-2-oxo-1,2,3,5-tetrahydro-4,1-benzoxazepine-3-acetic acid, (3R,5S)-7-cyano-5-(2,3-ethylenedioxyphenyl)-1-neopentyl-2-oxo-1,2,3,5-tetrahydro-4,1-benzoxazepine-3-acetic acid, (3R,5S)-7-cyano-5-(2,3-dimethoxyphenyl)-1-isobutyl-2-oxo-1,2,3,5-tetrahydro-4,1-benzoxazepine-3-acetic acid, (3R,5S)-7-cyano-5-(2,4-dimethoxyphenyl)-1-isobutyl-2-oxo-1,2,3,5-tetrahydro-4,1-benzoxazepine-3-acetic acid, (3R,5S)-7-cyano-5-(2,3-methylenedioxyphenyl)-1-isobutyl-2-oxo-1,2,3,5-tetrahydro-4,1-benzoxazepine-3-acetic acid, (3R,5S)-7-cyano-5-(2,3-ethylenedioxyphenyl)-1-isobutyl-2-oxo-1,2,3,5-tetrahydro-4,1-benzoxazepine-3-acetic acid, (3R,5S)-7-chloro-5-(2,3-dimethoxyphenyl)-1-neopentyl-2-oxo-1,2,3,5-tetrahydro-4,1-benzoxazepine-3-acetic acid, (3R,5S)-7-chloro-5-(2,4-dimethoxyphenyl)-1-neopentyl-2-oxo-1,2,3,5-tetrahydro-4,1-benzoxazepine-3-acetic acid, (3R,5S)-7-chloro-5-(2,3-methylenedioxyphenyl)-1-neopentyl-2-oxo-1,2,3,5-tetrahydro-4,1-benzoxazepine-3-acetic acid, (3R,5S)-7-chloro-5-(2,3-ethylenedioxyphenyl)-1-neopentyl-2-oxo-1,2,3,5-tetrahydro-4,1-benzoxazepine-3-acetic acid, (3R,5S)-7-chloro-5-(2,3-dimethoxyphenyl)-1-isobutyl-2-oxo-1,2,3,5-tetrahydro-4,1-benzoxazepine-3-acetic acid, (3R,5S)-7-chloro-5-(2,4-dimethoxyphenyl)-1-isobutyl-2-oxo-1,2,3,5-tetrahydro-4,1-benzoxazepine-3-acetic acid, (3R,5S)-7-chloro-5-(2,3-methylenedioxyphenyl)-1-isobutyl-2-oxo-1,2,3,5-tetrahydro-4,1-benzoxazepine-3-acetic acid, (3R,5S)-7-chloro-5-(2,3-ethylenedioxyphenyl)-1-isobutyl-2-oxo-1,2,3,5-tetrahydro-4,1-benzoxazepine-3-acetic acid, (3R,5S)-7-cyano-5-(2,3-dimethoxyphenyl)-1-neopentyl-2-oxo-1,2,3,5-tetrahydro-4,1-benzothiazepine-3-acetic acid, (3R,5S)-7-cyano-5-(2,4-dimethoxyphenyl)-1-neopentyl-2-oxo-1,2,3,5-tetrahydro-4,1-benzothiazepine-3-acetic acid, (3R,5S)-7-cyano-5-(2,3-methylenedioxyphenyl)-1-neopentyl-2-oxo-1,2,3,5-tetrahydro-4,1-benzothiazepine-3-acetic acid, (3R,5S)-7-cyano-5-(2,3-ethylenedioxyphenyl)-1-neopentyl-2-oxo-1,2,3,5-tetrahydro-4,1-benzothiazepine-3-acetic acid, (3R,5S)-7-cyano-5-(2,3-dimethoxyphenyl)-1-isobutyl-2-oxo-1,2,3,5-tetrahydro-4,1-benzothiazepine-3-acetic acid, (3R,5S)-7-cyano-5-(2,4-dimethoxyphenyl)-1-isobutyl-2-oxo-1,2,3,5-tetrahydro-4,1-benzothiazepine-3-acetic acid, (3R,5S)-7-cyano-5-(2,3-methylenedioxyphenyl)-1-isobutyl-2-oxo-1,2,3,5-tetrahydro-4,1-benzothiazepine-3-acetic acid, (3R,5S)-7-cyano-5-(2,3-ethylenedioxyphenyl)-1-isobutyl-2-oxo-1,2,3,5-tetrahydro-4,1-benzothiazepine-3-acetic acid, (3R,5S)-7-chloro-5-(2,3-dimethoxyphenyl)-1-neopentyl-2-oxo-1,2,3,5-tetrahydro-4,1-benzothiazepine-3-acetic acid, (3R,5S)-7-chloro-5-(2,4-dimethoxyphenyl)-1-neopentyl-2-oxo-1,2,3,5-tetrahydro-4,1-benzothiazepine-3-acetic acid, (3R,5S)-7-chloro-5-(2,3-methylenedioxyphenyl)-1-neopentyl-2-oxo-1,2,3,5-tetrahydro-4,1-benzothiazepine-3-acetic acid, (3R,5S)-7-chloro-5-(2,3-ethylenedioxyphenyl)-1-neopentyl-2-oxo-1,2,3,5-tetrahydro-4,1-benzothiazepine-3-acetic acid, (3R,5S)-7-chloro-5-(2,3-dimethoxyphenyl)-1-isobutyl-2-oxo-1,2,3,5-tetrahydro-4,1-benzothiazepine-3-acetic acid, (3R,5S)-7-chloro-5-(2,4-dimethoxyphenyl)-1-isobutyl-2-oxo-1,2,3,5-tetrahydro-4,1-benzothiazepine-3-acetic acid, (3R,5S)-7-chloro-5-(2,3-methylenedioxyphenyl)-1-isobutyl-2-oxo-1,2,3,5-tetrahydro-4,1-benzothiazepine-3-acetic acid, (3R,5S)-7-chloro-5-(2,3-ethylenedioxyphenyl)-1-isobutyl-2-oxo-1,2,3,5-tetrahydro-4,1-benzothiazepine-3-acetic acid, (3R,5S)-7-cyano-5-(2-chlorophenyl)-1-neopentyl-2-oxo-1,2,3,5-tetrahydro-4,1-benzoxazepine-3-acetic acid, (3R,5S)-7-cyano-5-(2-chlorophenyl)-1-isobutyl-2-oxo-1,2,3,5-tetrahydro-4,1-benzoxazepine-3-acetic acid, (3R,5S)-7-chloro-5-(2-chlorophenyl)-1-neopentyl-2-oxo-1,2,3,5-tetrahydro-4,1-benzoxazepine-3-acetic acid, (3R,5S)-7-chloro-5-(2-chlorophenyl)-1-isobutyl-2-oxo-1,2,3,5-tetrahydro-4,1-benzoxazepine-3-acetic acid, (3R,5S)-7-cyano-5-(2-chlorophenyl)-1-neopentyl-2-oxo-1,2,3,5-tetrahydro-4,1-benzothiazepine-3-acetic acid, (3R,5S)-7-cyano-5-(2-chlorophenyl)-1-isobutyl-2-oxo-1,2,3,5-tetrahydro-4,1-benzothiazepine-3-acetic acid, (3R,5S)-7-chloro-5-(2-chlorophenyl)-1-neopentyl-2-oxo-1,2,3,5-tetrahydro-4,1-benzothiazepine-3-acetic acid, (3R,5S)-7-chloro-5-(2-chlorophenyl)-1-isobutyl-2-oxo-1,2,3,5-tetrahydro-4,1-benzothiazepine-3-acetic acid, (3R,5S)-7-chloro-5-(4-ethoxy-2-methoxyphenyl)-1-neopentyl-2-oxo-1,2,3,5-tetrahydro-4,1-benzoxazepine-3-acetic acid, (3R)-7-chloro-5-(2-chlorophenyl)-2,3-dihydro-1-isobutyl-2-oxo-1H-1,4-benzodiazepine-3-acetic acid, (3R,5S)-7-chloro-5-(2-chlorophenyl)-2,3,4,5-tetrahydro-1-isobutyl-2-oxo-lH-1,4-benzodiazepine-3-acetic acid, N-[[(3R,5S)-7-chloro-5-(2-chlorophenyl)-1-neopentyl-2-oxo-1,2,3,5-tetrahydro-4,1-benzothiazepin-3-yl]-acetyl]glycine, (3R,5S)-7-chloro-5-(2-chlorophenyl)-3-dimethylaminocarbonylmethyl-1-neopentyl-2-oxo-1,2,3,5-tetrahydro-4,1-benzothiazepine, 7-chloro-5-(2-chlorophenyl)-1-isobutyl-2-oxo-2,3,4,5-tetrahydro-1H-[1]-benzazepine-3-acetic acid, (3R,5S)-7-chloro-5-(2-chlorophenyl)-1-neopentyl-1,2,3,5-tetrahydro-2-thioxo-4,1-benzoxazepine-3-acetic acid, (3R,5S)-7-chloro-5-(2-chlorophenyl)-1-neopentyl-2-oxo-1,2,3,5-tetrahydro-4,1-thieno[2,3-e]oxazepine-3-acetic acid, (3R,5S)-7-chloro-5-(2-methoxyphenyl)-1-neopentyl-2-oxo-1,2,3,5-tetrahydro-4,1-thieno[2,3-e]oxazepine-3-acetic acid, (3R,5S)-7-chloro-1-isobutyl-5-(2-methoxyphenyl)-2-oxo-1,2,3,5-tetrahydro-4,1-thieno[2,3-e]oxazepine-3-acetic acid, (3R,5S)-7-chloro-5-(3-hydroxy-2-methoxyphenyl)-1-neopentyl-2-oxo-1,2,3,5-tetrahydro-4,1-benzothiazepine-3-acetic acid, (3R,5S)-7-chloro-5-(4-hydroxy-2-methoxyphenyl)-1-neopentyl-2-oxo-1,2,3,5-tetrahydro-4,1-benzothiazepine-3-acetic acid, (3R,5S)-7-chloro-5-(3-hydroxy-2-methoxyphenyl)-1-isobutyl-2-oxo-1,2,3,5-tetrahydro-4,1-benzothiazepine-3-acetic acid, (3R,5S)-7-chloro-5-(4-hydroxy-2-methoxyphenyl)-1-isobutyl-2-oxo-1,2,3,5-tetrahydro-4,1-benzothiazepine-3-acetic acid, (3R,5S)-7-chloro-5-(3-ethoxy-2-methoxyphenyl)-1-neopentyl-2-oxo-1,2,3,5-tetrahydro-4,1-benzothiazepine-3-acetic acid, (3R,5S)-7-chloro-5-(4-ethoxy-2-methoxyphenyl)-1-neopentyl-2-oxo-1,2,3,5-tetrahydro-4,1-benzothiazepine-3-acetic acid, (3R,5S)-7-chloro-5-(3-ethoxy-2-methoxyphenyl)-1-isobutyl-2-oxo-1,2,3,5-tetrahydro-4,1-benzothiazepine-3-acetic acid, (3R,5S)-7-chloro-5-(4-ethoxy-2-methoxyphenyl)-1-isobutyl-2-oxo-1,2,3,5-tetrahydro-4,1-benzothiazepine-3-acetic acid, (3R,5S)-7-chloro-5-(2-chloro-3-methoxyphenyl)-1-neopentyl-2-oxo-1,2,3,5-tetrahydro-4,1-benzothiazepine-3-acetic acid, (3R,5S)-7-chloro-5-(2-chloro-4-methoxyphenyl)-1-neopentyl-2-oxo-1,2,3,5-tetrahydro-4,1-benzothiazepine-3-acetic acid, (3R,5S)-7-chloro-5-(2-chloro-3-methoxyphenyl)-1-isobutyl-2-oxo-1,2,3,5-tetrahydro-4,1-benzothiazepine-3-acetic acid, (3R,5S)-7-chloro-5-(2-chloro-4-methoxyphenyl)-1-isobutyl-2-oxo-1,2,3,5-tetrahydro-4,1-benzothiazepine-3-acetic acid, (3R,5S)-7-chloro-5-(2-chloro-3-hydroxyphenyl)-1-neopentyl-2-oxo-1,2,3,5-tetrahydro-4,1-benzothiazepine-3-acetic acid, (3R,5S)-7-chloro-5-(2-chloro-4-hydroxyphenyl)-1-neopentyl-2-oxo-1,2,3,5-tetrahydro-4,1-benzothiazepine-3-acetic acid, (3R,5S)-7-chloro-5-(2-chloro-3-hydroxyphenyl)-1-isobutyl-2-oxo-1,2,3,5-tetrahydro-4,1-benzothiazepine-3-acetic acid, (3R,5S)-7-chloro-5-(2-chloro-4-hydroxyphenyl)-1-isobutyl-2-oxo-1,2,3,5-tetrahydro-4,1-benzothiazepine-3-acetic acid, and salts thereof, and the like.

The above-mentioned compounds represented by the general formula (I) and salts thereof [hereinafter sometimes referred to merely as compound (I), including a salt thereof] are disclosed in EPA567026, WO95/21834 (Japanese Patent Application No. H6-15531), EPA645377 (Japanese Patent Application No. H6-229159), EPA645378 (Japanese Patent Application No. H6-229160) and the like, and may be prepared according to the disclosure of these publications.

As the compound of the formula (I), a compound represented by the above-mentioned formula (Ib) 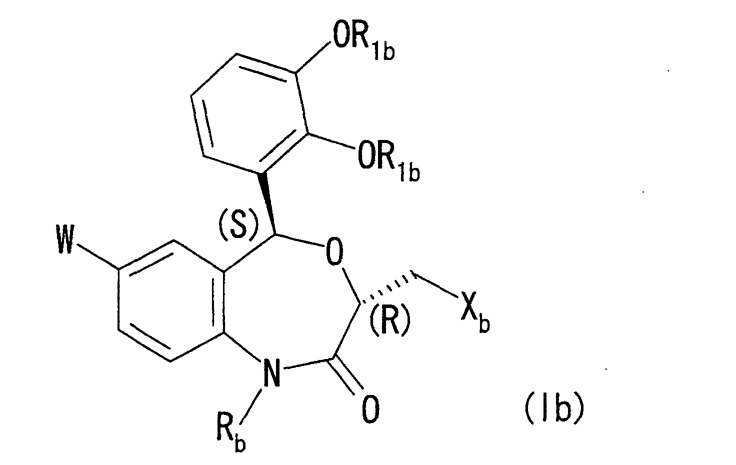 is preferred.

As the compound of the formula (Ib), a compound wherein R_{b} is a C₁₋₆ alkyl optionally having 1 to 3 substituents selected from a hydroxy group, an acetyloxy, a propionyloxy, a t-butoxycarbonyloxy, a palmitoyloxy, a dimethylaminoacetyloxy and a 2-aminopropionyloxy;
a compound wherein R_{b} is a branched C₃₋₆ alkyl optionally having 1 to 3 substituents selected from a hydroxy group, an acetyloxy, a propionyloxy, a t-butoxycarbonyloxy, a palmitoyloxy, a dimethylaminoacetyloxy and a 2-aminopropionyloxy;
a compound wherein R_{b} is a 2,2-dimethyl-3-hydroxypropyl, a 3-hydroxy-2-hydroxymethyl-2-methylpropyl, a 3-acetoxy-2,2-dimethylpropyl, a 3-acetoxy-2-hydroxymethyl-2-methylpropyl or a 3-acetoxy-2-acetoxymethyl-2-methylpropyl;
a compound wherein R_{1b} is a methyl;
a compound wherein W is a chlorine atom;
a compound wherein X_{b} is a group represented by the formula 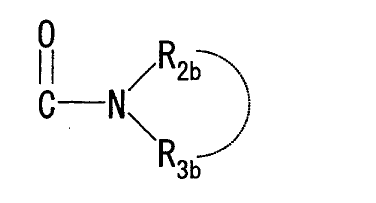 wherein R_{2b} and R_{3b} are each a hydrogen atom, an optionally substituted hydrocarbon group, an optionally substituted heterocyclic group or an acyl group, or R_{2b} and R_{3b} may form, together with the adjacent nitrogen atom, an optionally substituted 5- or 6-membered nitrogen-containing heterocyclic ring which may have 1 to 3 heteroatoms selected from a nitrogen atom, a sulfur atom and an oxygen atom as the ring-constituting atoms;
a compound wherein
in the group represented by X_{b},
R_{2b} is a hydrogen atom or a C₁₋₇ alkyl group,
R_{3b} is
   (1) a hydrocarbon group selected from (a) a C₁₋₇ alkyl, (b) a C₃₋₇ cycloalkyl, (c) a C₂₋₆ alkenyl , (d) a C₆₋₁₀ aryl and (e) a C₆₋₁₀ aryl-C₁₋₄ alkyl
      [wherein each (a) a C₁₋₇ alkyl, (b) a C₃₋₇ cycloalkyl and (c) a C₂₋₆ alkenyl optionally may have 1 to 4 substituents selected from
      (i) a carboxyl group optionally esterified with a C₁₋₆ alkyl or a C₆₋₁₀ aryl-C₁₋₄ alkyl,
      (ii) a phosphoric acid group optionally mono- or di-substituted with a C₁₋₆ alkyl or a C₂₋₇ alkanoyloxy-C₁₋₆ alkyl,
      (iii) a sulfonic acid group,
      (iv) a sulfonamide group optionally substituted with a C₁₋₆ alkyl or a C₆₋₁₀ aryl-C₁₋₄ alkyl,
      (v) a hydroxy group optionally alkylated with a C₁₋₃ alkyl,
      (vi) a sulfhydryl group optionally alkylated with a C₁₋₃ alkyl,
      (vii) a carbamoyl group,
      (viii) a phenyl group optionally substituted with 1 to 5 substituents selected from a hydroxy group, a chlorine atom, a fluorine atom, an aminosulfonyl and an amino group optionally mono- or di-substituted with a C₁₋₃ alkyl,
      (ix) an amino group optionally mono- or di-substituted with a C₁₋₃ alkyl,
      (x) a cyclic amino group optionally substituted with a C₁₋₃ alkyl, a benzyl or a phenyl, which is derived from a piperidine, a pyrrolidine, a morpholine, a thiomorpholine, a piperazine, a 4-methylpiperazine, a 4-benzylpiperazine, a 4-phenylpiperazine, a 1,2,3,4-tetrahydroisoquinoline or a phthalimide, and
      (xi) an aromatic 5 to 6-membered heterocyclic group derived from a pyridine, an imidazol, an indol or a tetrazol,
      each (d) a C₆₋₁₀ aryl and (e) a C₆₋₁₀ aryl-C₁₋₄ alkyl may have 1 to 4 substituents selected from
      (i) a carboxyl group optionally esterified with a C₁₋₄ alkyl,
      (ii) a phosphoric acid group optionally mono- or di-substituted with a C₁₋₆ alkyl or a C₂₋₇ alkanoyloxy-C₁₋₆ alkyl,
      (iii) a sulfonic acid group,
      (iv) a C₁₋₄ alkylsulfonyl, a C₆₋₁₀ arylsulfonyl or a C₆₋₁₀ aryl-C₁₋₄ alkylsulfonyl group,
      (v) a sulfonamide group optionally substituted with a C₁₋₆ alkyl or a C₆₋₁₀ aryl-C₁₋₄ alkyl,
      (vi) a C₁₋₃ alkyl group optionally substituted with a carboxyl group which may be esterified with a C₁₋₄ alkyl, a phosphoric acid group which may be mono- or di- substituted with a C₁₋₆ alkyl, a sulfonic acid group, a C₁₋₄ alkylsulfonyl, a C₆₋₁₀ arylsulfonyl or a C₆₋₁₀ aryl-C₁₋₄ alkylsulfonyl group, a sulfonamide group which may be substituted with a C₁₋₆ alkyl or a C₆₋₁₀ aryl-C₁₋₄ alkyl, and
      (vii) a halogen],
   (2) a tetrazolyl, a 4,5-dihydro-5-oxo-1,2,4-oxadiazolyl, a 4,5-dihydro-5-thioxo-1,2,4-oxadiazolyl, a 2,3-dihydro-3-oxo-1,2,4-oxadiazolyl, a 2,3-dihydro-3-thioxo-1,2,4-oxadiazolyl, a 3,5-dioxo-1,2,4-oxadiazolidinyl, a 4,5-dihydro-5-oxo-isoxazolyl, a 4,5-dihydro-5-thioxo-isoxazolyl, a 2,3-dihydro-2-oxo-1,3,4-oxadiazolyl, a 2,3-dihydro-3-oxo-1,2,4-tetrazolyl or a 2,3-dihydro-3-thioxo-1,2,4-tetrazolyl group, or
   (3) an acyl group selected from (i) a C₂₋₇ alkanoyl group optionally substituted with 1 or 2 halogen(s) and (ii) a C₆₋₁₀ arylsulfonyl group, a C₁₋₄ alkylsulfonyl group or a C₆₋₁₀ aryl-C₁₋₄ alkylsulfonyl group, each of which is optionally substituted with 1 to 4 substituents selected from a C₁₋₃ alkyl, a C₁₋₃ alkoxy and a halogen or
   R_{2b} and R_{3b} form, together with the adjacent nitrogen atom, a 5-membered or 6-membered ring derived from piperidine, piperazine, pyrrolidine, 2-oxopiperazine, 2,6-dioxopiperazine, morpholine or thiomorpholine [wherein the 5-membered or 6-membered ring may have 1 to 4 substituents selected from
   (A) a hydroxy group optionally substituted with a C₁₋₃ alkyl or a C₂₋₇ alkanoyl,
   (B) a carboxyl group optionally esterified with a C₁₋₆ alkyl or a C₆₋₁₀ aryl-C₁₋₄ alkyl,
   (C) a phosphoric acid group optionally mono- or di-substituted with a C₁₋₆ alkyl or a C₂₋₇ alkanoyloxy-C₁₋₆ alkyl,
   (D) a sulfonic acid group,
   (E) a sulfonamide group optionally substituted with a C₁₋₆ alkyl or a C₆₋₁₀ aryl-C₁₋₄ alkyl,
   (F) a C₁₋₆ alkyl and a C₂₋₅ alkenyl, each of which is optionally substituted with:
      a carboxyl group optionally esterified with a C₁₋₆ alkyl or a C₆₋₁₀ aryl-C₁₋₄ alkyl,
      a phosphoric acid group optionally mono- or di-substituted with a C₁₋₆ alkyl or a C₂₋₇ alkanoyloxy-C₁₋₆ alkyl,
      a sulfonic acid group,
      a sulfonamide group optionally substituted with a C₁₋₆ alkyl or a C₆₋₁₀ aryl-C₁₋₄ alkyl,
      a hydroxy group optionally substituted with a C₁₋₃ alkyl or C₂₋₇ alkanoyl,
      a sulfhydryl group optionally alkylated with a C₁₋₃ alkyl,
      a carbamoyl group,
      a phenyl group optionally substituted with 1 to 5
      substituents selected from a hydroxy group, a halogen, an aminosulfonyl and an amino group optionally substituted with a C₁₋₃ alkyl,
      an amino group optionally mono- or di- substituted with a C₁₋₃ alkyl, or
      a tetrazolyl group,
   (G) an amino group optionally mono- or di- substituted with a C₁₋₃ alkyl,
   (H) a cyclic amino group derived from a piperidine, a pyrrolidine, a morpholine, a thiomorpholine, a 4-methylpiperazine, a 4-benzylpiperazine or a 4-phenylpiperazine,
   (I) a cyano group,
   (J) a carbamoyl group,
   (K) an oxo group,
   (L) a tetrazolyl or a 2,5-dihydro-5-oxo-1,2,4-oxadiazolyl group,
   (M) a carbamoyl group optionally substituted with a C₆₋₁₀ arylsulfonyl, a C₁₋₄ alkylsulfonyl or a C₆₋₁₀ aryl-C₁₋₄ alkylsulfonyl,
   (N) a sulfhydryl group optionally alkylated with a C₁₋₃ alkyl, and
   (O) a phenyl group optionally substituted with a hydroxy group, a halogen, an aminosulfonyl and an amino group optionally substituted with a C₁₋₃ alkyl]; a compound wherein, in the group represented by X_{b},
R_{2b} and R_{3b} form, together with the nitrogen atom of the carbamoyl group, a 5-membered or 6-membered ring derived from a piperidine, a piperazine, a pyrrolidine, a 2-oxopiperazine or a 2,6-dioxopiperazine, each of the 5-membered or 6-membered ring is optionally substituted with a C₁₋₆ alkyl group optionally having 1 or 2 substituents selected from
   (i) a carboxyl group optionally esterified with a C₁₋₆ alkyl or a C₆₋₁₀ aryl-C₁₋₄ alkyl,
   (ii) a phosphoric acid group optionally mono- or di-substituted with a C₁₋₆ alkyl or a C₂₋₇ alkanoyl-C₁₋₆ alkyl,
   (iii) a sulfonic acid group,
   (iv) a sulfonamide group optionally substituted with a C₁₋₆ alkyl or a C₆₋₁₀ aryl-C₁₋₄ alkyl,
   (v) a hydroxy group optionally alkylated with a C₁₋₃ alkyl,
   (vi) a sulfhydryl group optionally alkylated with a C₁₋₃ alkyl,
   (vii) a carbamoyl group,
   (viii) a phenyl group optionally substituted with 1 to 5 substituents selected from a hydroxy group, a halogen, an aminosulfonyl and an amino group optionally substituted with a C₁₋₃ alkyl,
   (ix) an amino group optionally mono- or di- substituted with a C₁₋₃ alkyl,
      and
   (x) a tetrazolyl group;
a compound wherein, in the group represented by X_{b}, R_{2b} is a hydrogen atom or a C₁₋₇ alkyl, and R_{3b} is a C₁₋₄ alkylsulfonyl;
a compound wherein the heterocyclic group represented by X_{b} is a tetrazolyl, a 4,5-dihydro-5-oxo-1,2,4-oxadiazolyl, a 4,5-dihydro-5-thioxo-1,2,4-oxadiazolyl, a 2,3-dihydro-3-oxo-1,2,4-oxadiazolyl, a 2,3-dihydro-3-thioxo-1,2,4-oxadiazolyl, a 3,5-dioxo-1,2,4-oxadiazolidinyl, a 4,5-dihydro-5-oxo-isoxazolyl, a 4,5-dihydro-5-thioxo-isoxazolyl, a 2,3-dihydro-2-oxo-1,3,4-oxadiazolyl, a 2,3-dihydro-3-oxo-1,2,4-tetrazolyl or a 2,3-dihydro-3-thioxo-1,2,4-tetrazolyl;
a compound wherein
   R_{1b} is a methyl,
   W is a chlorine atom,
   R_{b} is a branched C₃₋₆ alkyl substituted with 1 to 3 substituents selected from a hydroxy group, an acetyloxy, a propionyloxy, a tert-butoxycarbonyloxy, a palmitoyloxy, a dimethylaminoacetyloxy and a 2-aminopropionyloxy,
   X_{b} is a group represented by the formula 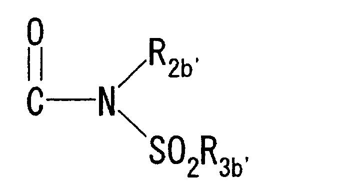 wherein R_{2b}, is a hydrogen atom or a C₁₋₇ alkyl and R_{3b}, is a C₁₋₄ alkyl;
a compound wherein
   R_{1b} is a methyl,
   W is a chlorine atom,
   R_{b} is a branched C₃₋₆ alkyl substituted with 1 to 3 substituents selected from a hydroxy group, an acetyloxy, a propionyloxy, a tert-butoxycarbonyloxy, a palmitoyloxy, a dimethylaminoacetyloxy and a 2-aminopropionyloxy,
   X_{b} is a group represented by the formula: 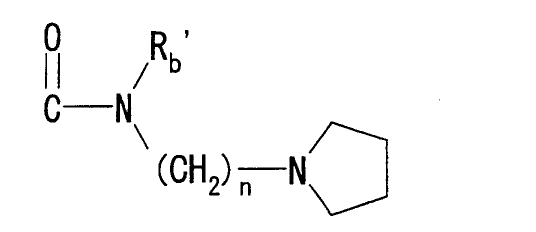 wherein R_{b}' is a hydrogen atom or a C₁₋₇ alkyl and n is an integer of 1 to 5;
a compound wherein
   R_{1b} is a methyl,
   W is a chlorine atom,
   R_{b} is a branched C₃₋₆ alkyl substituted with 1 to 3 substituents selected from a hydroxy group, a acetyloxy, a propionyloxy, a tert-butoxycarbonyloxy, a palmitoyloxy, a dimethylaminoacetyloxy and a 2-aminopropionyloxy,
   X_{b} is a group represented by the formula: 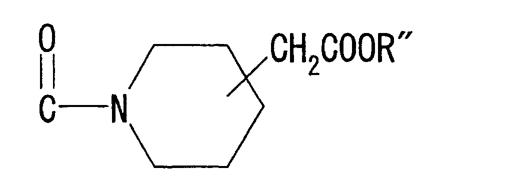 wherein R" is a hydrogen atom or a C₁₋₄ alkyl;
a compound wherein
   R_{1b} is a methyl,
   W is a chlorine atom,
   R_{b} is a branched C₃₋₆ alkyl substituted with 1 to 3 substituents selected from a hydroxy group, a acetyloxy, a propionyloxy, a tert-butoxycarbonyloxy, a palmitoyloxy, a dimethylaminoacetyloxy and a 2-aminopropionyloxy, and
   X_{b} is tetrazolyl;
a compound wherein
   R_{b} is a lower alkyl optionally substituted with 1 or 2 hydroxy groups,
   X_{b} is
      (1) a carbamoyl group optionally substituted with a hydrocarbon group selected from (a) a C₁₋₇ alkyl, (b) a C₃₋₇ cycloalkyl, (c) a C₂₋₆ alkenyl , (d) a C₆₋₁₀ aryl and (e) a C₇₋₁₄ arylalkyl
         [wherein each (a) a C₁₋₇ alkyl, (b) a C₃₋₇ cycloalkyl, (c) a C₂₋₆ alkenyl may have 1 to 4 substituents selected from
         (i) a carboxyl group optionally esterified with a C₁₋₆ alkyl or a C₇₋₁₀ arylalkyl,
         (ii) a phosphoric acid group,
         (iii) a sulfonic acid group,
         (iv) a sulfonamide group optionally substituted with a C₁₋₆ alkyl or a C₇₋₁₀ arylalkyl,
         (v) a hydroxy group optionally alkylated with a C₁₋₃ alkyl,
         (vi) a sulfhydryl group optionally alkylated with a C₁₋₃ alkyl,
         (vii) a carbamoyl group,
         (viii) a phenyl group optionally substituted with substituents selected from a hydroxy group, a chlorine atom, a fluorine atom, an aminosulfonyl and an amino group optionally mono- or di- substituted with a C₁₋₃ alkyl,
         (ix) an amino group optionally mono- or di- substituted with a C₁₋₃ alkyl, and
         (x) a cyclic amino group optionally substituted with a C₁₋₃ alkyl, a benzyl or a phenyl, which is derived from a piperidine, a pyrrolidine, a morpholine, a thiomorpholine, a piperazine, a 4-methylpiperazine, a 4-benzylpiperazine or a 4-phenylpiperazine, and
         (xi) an aromatic 5 to 6-membered heterocyclic group derived from a pyridine, an imidazol, an indol or a tetrazol, each (d) a C₆₋₁₀ aryl and (e) a C₇₋₁₄ arylalkyl may have 1 to 4 substituents selected from

         (i) a carboxyl group optionally esterified with C₁₋₄ alkyl,
         (ii) a phosphoric acid group,
         (iii) a sulfonic acid group,
         (iv) a sulfonamide group optionally substituted with a C₁₋₆ alkyl or a C₇₋₁₀ arylalkyl,
         (v) a C₁₋₃ alkyl group optionally substituted with a carboxyl group optionally esterified with a C₁₋₄ alkyl, a phosphoric acid group, a sulfonic acid group, a sulfonamide group optionally substituted with a C₁₋₆ alkyl or a C₇₋₁₀ arylalkyl,
            or
         (vi) a halogen atom],
      (2) a tetrazolyl, a 4,5-dihydro-5-oxo-1,2,4-oxadiazolyl, a 4,5-dihydro-5-thioxo-1,2,4-oxadiazolyl, a 2,3-dihydro-3-oxo-1,2,4-oxadiazolyl, a 2,3-dihydro-3-thioxo-1,2,4-oxadiazolyl, a 3,5-dioxo-1,2,4-oxadiazolidinyl, a 4,5-dihydro-5-oxo-isoxazolyl, a 4, 5-dihydro-5-thioxo-isoxazolyl, a 2,3-dihydro-2-oxo-1,3,4-oxadiazolyl, a 2,3-dihydro-3-oxo-1,2,4-tetrazolyl or a 2,3-dihydro-3-thioxo-1,2,4-tetrazolyl group,
      (3) a carbamoyl optionally substituted with an acyl group selected from
         (i) a C₂₋₇ alkanoyl group optionally substituted with 1 or 2 halogens and (ii) a C₆₋₁₀ arylsulfonyl group, a C₁₋₄ alkylsulfonyl group or a C₇₋₁₄ arylalkylsulfonyl group, each of which is optinally substituted with 1 to 4 substituents selected from a C₁₋₃ alkyl, a C₁₋₃ alkoxy and a halogen, , or
      (4) a cyclic aminocarbonyl group derived from a piperidine, a piperazine, a pyrrolidine, a 2-oxopiperazine, a 2,6-dioxopiperazine, a morpholine and a thiomorpholine [wherein the cyclic aminocarbonyl group may have 1 to 4 substituents selected from
         (A) a hydroxy group,
         (B) a carboxyl group optionally esterified with a C₁₋₄ alkyl,
         (C) a phosphoric acid group,
         (D) a sulfonic acid group,
         (E) a sulfonamide group optionally substituted with a C₁₋₆ alkyl or a C₇₋₁₀ arylalkyl,
         (F) a C₁₋₃ alkyl or a C₂₋₅ alkenyl optionally substituted with the above-mentioned (A), (B), (C), (D) or (E),
         (G) an amino group optionally mono- or di- substituted with a C₁₋₃ alkyl,
         (H) a cyclic amino group derived from a piperidine, a pyrrolidine, a morpholine, a thiomorpholine, a 4-methylpiperazine, a 4-benzylpiperazine or a 4-phenylpiperazine,
         (I) a cyano group,
         (J) a carbamoyl group,
         (K) an oxo,
         (L) a C₁₋₃ alkoxy,
         (M) a heterocyclic group derived from a tetrazolyl or a 2,5-dihydro-5-oxo-1,2,4-oxadiazolyl, and
         (N) a carbamoyl group optionally substituted with a C₆₋₁₀ arylsulfonyl, a C₁₋₄ alkylsulfonyl or a C₇₋₁₄ arylalkylsulfonyl];
   a compound wherein R_{b} is a 2,2-dimethyl-3-hydroxypropyl group;
and the like are preferred.

In the above-mentioned formula, the lower alkyl group represented by R_{b} includes a C₁₋₆ alkyl such as methyl, ethyl, n-propyl, isopropyl, n-butyl, isobutyl, n-pentyl, isopentyl, neopentyl, hexyl and the like. Of these, a C₃₋₆ alkyl group is preferred, and a C₄₋₅ alkyl group is more preferred. Specifically, a branched C₄₋₅ alkyl group such as isobutyl, neopentyl and the like are preferred.

The substituents of the lower alkyl represented by R_{b} includes for example a hydroxy group optionally substituted with a C₂₋₂₀ alkanoyl or a C₁₋₇ alkyl and the like. Such substituents include for example a hydroxy group, acetyloxy, propionyloxy, tert-butoxycarbonyloxy, palmitoyloxy, dimethylaminoacetyloxy and 2-aminopropionyloxy and the like.

One to three of such substituents may be substituted at the substitutable positions.

Furthermore, the optionally substituted lower alkyl represented by R_{b} includes for example 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methyl-propyl and 3-acetoxy-2-acetoxymethyl-2-methylpropyl and the like.

The optionally substituted carbamoyl group reprsented by X_{b} includes a group represented by the formula 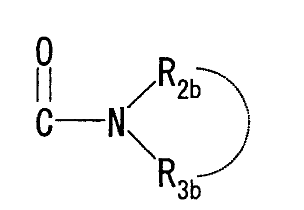 and the like.

The "optionally substituted hydrocarbon" represented by R_{2b} and R_{3b} includes such as an optionally substituted C₁₋ ₇ straight or branched alkyl group (e.g., methyl, ethyl, n-propyl, isopropyl, n-butyl, isobutyl, 1,1-dimethylethyl, n-pentyl, 3-methylbutyl, 2-methylbutyl, 1-methylbutyl, 1-ethylpropyl, n-hexyl, 4-methylpentyl, 3-methylpentyl, 2-methylpentyl, 2-ethylbutyl, 1-ethylbutyl, neopentyl, hexyl, heptyl), an optionally substituted C₃₋₇ cycloalkyl group (cyclopropyl, cyclobutyl, cyclopentyl, cyclohexyl, cyclohexylmethyl and the like), an optionally substituted C₂₋₆ straight or branched alkenyl group (e.g., vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like), an optionally substituted C₆₋₁₀ aryl group (e.g., phenyl, naphthyl group) and an optionally substituted C₇₋₁₄ arylalkyl group (e.g., benzyl, phenetyl, naphthylmethyl) and the like.

The substituents of the "optionally substituted C₁₋₇ straight or branched alkyl group, optionally substituted C₃₋₇ cycloalkyl group and C₂₋₆ straight or branched alkenyl group" include: a carboxyl group optionally esterified with a C₁₋₆ alkyl group or a C₆₋₁₀ aryl-C₁₋₄ alkyl group (e.g., methyl, ethyl, propyl, isopropyl, butyl, tert-butyl, phenyl, benzyl and the like); a phosphoric acid group optionally mono- or di- substituted with a C₁₋₆ alkyl (e.g., methyl, ethyl, n-propyl, isopropyl, n-butyl, isobutyl, n-pentyl, isopentyl, neopentyl, hexyl and the like) or a C₂₋₇ alkanoyloxy-C₁₋₆ alkyl such as acetyloxymethyl, pivaloyloxymethyl group; a sulfonic acid group; a sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl and the like); a hydroxy group and a sulfhydryl group, each of which is optionally alkylated with a C₁₋₃ alkyl group (e.g., methyl, ethyl, propyl and the like); a carbamoyl group; a phenyl group optionally substituted with 1 to 5 substituents [for example, a hydroxy group, a chlorine, a fluorine, an aminosulfonyl group, an amino group optionally substituted with a C₁₋₃ alkyl group (e.g., methyl, ethyl, propyl and the like)]; an amino group optionally mono- or di- substituted with a C₁₋₃ alkyl group (e.g., methyl, ethyl, propyl and the like); a cyclic amino group (e.g., a 5 to 6-membered cyclic amino group optionally substituted with a C₁₋₃ alkyl, benzyl, phenyl and the like, and optionally containing an oxygen atom, a sulfur atom as the ring-constituting atoms, which is derived from piperidine, pyrrolidine, morpholine, thiomorpholine, piperazine, 4-methylpiperazine, 4-benzylpiperazine, 4-phenylpiperazine, 1,2,3,4-tetrahydroisoquinoline, phthalimide and the like, and an aromatic 5 to 6-membered heterocyclic ring containing 1 to 4 heteroatoms selected from N, O, S (e.g., pyridine, imidazol, indol, tetrazol and the like) and the like.

Furthermore, the substituents of the C₆₋₁₀ aryl group and C₆₋₁₀ aryl-C₁₋₄ alkyl group as substituents of the optionally substituted amino group that forms the carbamoyl group of the "optionally substituted carbamoyl group" represented by X_{b}, include:
a carboxyl group optionally esterified with a C₁₋₄ alkyl group (methyl, ethyl, propyl, tert-butyl group and the like); a phosphoric acid group optionally mono- or di-substituted with a C₁₋₆ alkyl (methyl, ethyl, n-propyl, isopropyl, n-butyl, isobutyl, n-pentyl, isopentyl, neopentyl, hexyl) or a C₂₋₇ alkanoyloxy-C₁₋₆ alkyl group such as pivaloyloxymethyl group, acetyloxymethyl group and the like; a sulfonic acid group; a C₁₋₄ alkylsulfonyl, a C₆₋₁₀ arylsulfonyl or a C₆₋₁₀ aryl-C₁₋₄ alkylsulfonyl; a sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl and the like); and
a C₁₋₃ alkyl group optionally substituted with a carboxyl group optionally esterified with a C₁₋₄ alkyl group, a phosphoric acid group optionally mono- or di- substituted with a C₁₋₆ alkyl group such as methyl, ethyl, n-propyl, isopropyl, n-butyl, isobutyl, n-pentyl, isopentyl, neopentyl, hexyl and the like or a C₂₋₇ alkanoyloxy-C₁₋₆ alkyl group such as pivaloyloxymethyl group and the like, a sulfonic acid group and a sulfonamide group optionally substituted with a C₁₋₆ alkyl, a C₆₋₁₀ aryl-C₁₋₄ alkyl (e.g., methyl, ethyl, propyl, isopropyl);
a halogen (fluorine, chlorine), and the like.

The "hydrocarbon group" may have 1 to 5 substituents at the substitutable positions.

The "optionally substituted heterocyclic group" represented by R_{2b} and R_{3b} is preferably a heterocyclic group optionally having 1 or 2 (preferably one) substituents such as an oxo group, a thioxo group and the like and having a hydrogen atom that may be deprotonated. Such heterocyclic group is preferably a 5 to 6-membered heterocyclic group containing 1 to 4, preferably 2 to 3 heteroatoms selected from S, O, N. Specifically, tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolidinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl and 2,3-dihydro-3-thioxo-1,2,4-tetrazolyl and the like are exemplified. Specifically, tetrazolyl group is preferred.

The "acyl group" represented by R_{2b} and R_{3b} includes a carboxylic acid acyl group derived from carboxylic acid (e.g., a C₂₋₇ carboxylic acid acyl group such as acetyl, propionyl, butyryl, benzoyl and the like) and a C₆₋₁₀ arylsulfonyl group, a C₁₋₄ alkylsulfonyl group and a C₆₋₁₀ aryl-C₁₋₄ alkylsulfonyl group, each of which may have substituents (e.g., methylsulfonyl, ethylsulfonyl, phenylsulfonyl, naphthylsulfonyl, phenylmethylsulfonyl, phenylethylsulfonyl, naphthylmethylsulfonyl, naphthylethylsulfonyl and the like) and the like. The substituents of the aryl, alkyl- and arylalkylsulfonyl group include a C₁-C₃ alkyl (e.g., methyl, ethyl, propyl and the like), a C₁₋₃ alkoxy (e.g., methoxy, ethoxy, propoxy and the like), a halogen (chlorine, fluorine, bromine) and the like, and 1 to 4, preferably 1 or 2 of these substituents may be substituted at the substitutable positions.

The above-mentioned carboxylic acid acyl group may have 1 or 2 halogens (chlorine, fluorine, bromine) as a substituent.

The cyclic amino group optionally substituted with a C₁₋₃ alkyl or a C₂₋₇ alkanoyl and the like, which is formed by R_{2b} and R_{3b} with the adjacent nitrogen atom of carbamoyl, includes a group derived from a 5 or 6-membered cyclic amine optionally containing 1 to 3 heteroatoms selected from a nitrogen atom, a sulfur atom and an oxygen atom as the ring-constituting atoms, which is a cyclic amine such as piperazine, piperidine, pyrrolidine, piperazine-2-one, piperazine-2,6-dione, morpholine, thiomorpholine and the like. These cyclic amino groups may have 1 to 4, preferably 1 or 2 substituents. The substituents include a hydroxy group optionally substituted with a C₁₋₃ alkyl or a C₂₋₇ alkanoyl, a carboxyl group optionally esterified with a C₁₋₄ alkyl group (methyl, ethyl, propyl, tert-butyl group and the like) or a C₇₋₁₀ arylalkyl, a phosphoric acid group optionally mono- or di- substituted with a C₁₋₆ alkyl or a C₂₋₇ alkanoyloxy-C₁₋₆ alkyl group (acetyloxymethyl group, pivaloyloxymethyl group); a sulfonic acid group; a sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl and the like); a C₁₋₆ alkyl and a C₂₋₅ alkenyl, each of which is optionally substituted with "a carboxyl group optionally esterified with a C₁₋₆ alkyl or a C₆₋₁₀ aryl-C₁₋₄ alkyl; a phosphoric acid group optionally mono- or di- substituted with a C₁₋₆ alkyl or a C₂₋₇ alkanoyloxy-C₁₋₆ alkyl group (acetyloxymethyl group, pivaloyloxymethyl group and the like); a sulfonic acid group; a sulfonamide group optionally substituted with a C₁₋₆ alkyl or a C₆₋₁₀ aryl-C₁₋₄ alkyl; a hydroxy group optionally substituted with a C₁₋₃ alkyl or a C₂₋₇ alkanoyl; a sulfhydryl group optionally alkylated with a C₁₋₃ alkyl; a carbamoyl group; a phenyl optionally substituted with 1 to 5 substituents (e.g., a hydroxy group, a halogen, an aminosulfonyl, an amino group optionally substituted with a C₁₋₃ alkyl and the like); an amino group optionally mono- or di- substituted C₁₋₃ alkyl; or a tetrazolyl"; an amino group optionally mono- or di- substituted with a C₁₋₃ alkyl group (e.g., methyl, ethyl, propyl and the like); a cyclic amino group (e.g., a group derived from a 5-or 6- membered cyclic amine optionally containing heteroatom(s) selected from a nitrogen atom, a sulfur atom and an oxygen atom, and optionally substituted with a C₁-C₃ alkyl, a benzyl, a phenyl, such as piperidine, pyrrolidine, morpholine, thiomorpholine, 4-methylpiperazine, 4-benzylpiperazine, 4-phenylpiperazine and the like); a cyano group; a carbamoyl group; an oxo group; a C₁₋₃ alkoxy (e.g., methoxy, ethoxy, ethylenedioxy and the like); a heterocyclic group optionally substituted with an oxo group or a thioxo group and having a hydrogen atom that may be deprotonated as mentioned above (e.g., tetrazolyl, 2,5-dihydro-5-oxo-1,2,4-oxadiazolyl and the like), a carbamoyl group substituted with a C₆₋₁₀ arylsulfonyl, a C₆₋₁₀ aryl-C₁₋₄ alkylsulfonyl and a C₁₋₄ alkylsulfonyl (methylsulfonyl, ethylsulfonyl, propylsulfonyl, butylsulfonyl, isopropylsulfonyl, tert-butylsulfonyl, phenyl, sulfonyl, benzylsulfonyl and the like), a sulfhydryl group optionally substituted with a C₁₋₃ alkyl or a phenyl group optionally substituted with 1 to 5 substituents (e.g., a hydroxy group, a halogen, an aminosulfonyl and an amino optionally substituted with a C₁₋₃ alkyl and the like), which are exemplified as the substituents of the optionally substituted amino that forms the carbamoyl of the "optionally substituted carbamoyl group" represented by X), and the like.

Examples of the optionally substituted carbamoyl group represented by X_{b} include 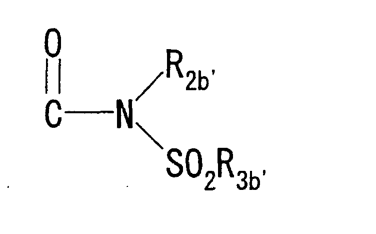 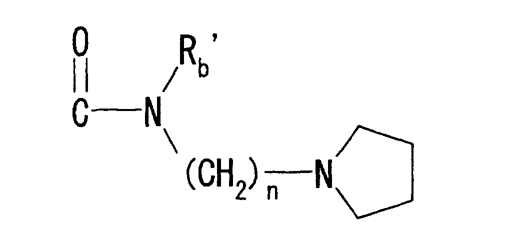 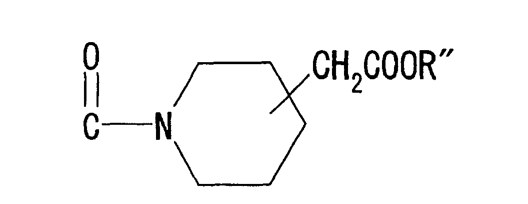 and the like.

R_{2b}, and R_{b}, include a hydrogen atom and a C₁₋₇ alkyl and the like. Specifically, a hydrogen atom is preferred.

The C₁₋₇ alkyl represented by R_{2b}, R_{2b}, and R_{b}' includes those exemplified as the C₁₋₇ alkyl of the above-mentioned "hydrocarbon group".

R" includes a hydrogen atom and a C₁₋₄ alkyl and the like. Of these, a hydrogen atom is preferred.

The C₁₋₄ alkyl represented by R_{3b}, and R" include for example methyl, ethyl, propyl, isopropyl, n-butyl, tert-butyl and the like.

As the optionally substituted heterocyclic group having a hydrogen atom that may be deprotonated, represented by X_{b}, a nitrogen-containing 5 to 6-membered heterocyclic ring (preferably containing 1 to 4 nitrogen atoms) having an active proton like a Bronsted acid is preferred, and a preferred heterocyclic group contains 1 to 4, preferably 2 to 3 of a nitrogen atom, a sulfur atom or an oxygen atom. As the substituents of the group, an oxo group, a thioxo group and the like are exemplified, and the heterocyclic group may have 1 or 2, specifically one of these substituents. The "optionally substituted heterocyclic group having a hydrogen atom that may be deprotonated" represented by X includes those exemplified as the "optionally substituted heterocyclic group" as substituents of the "optionally substituted carbamoyl group" represented by X, such as a tetrazolyl, a 2,5-dihydro-5-oxo-1,2,4-oxadiazolyl and the like.

The "lower alkyl group" represented by R_{1b} include a C₁-C₆ alkyl group such as methyl, ethyl, n-propyl, isopropyl, n-butyl, tert-butyl, pentyl, hexyl and the like. Of these, a C₁-C₃ alkyl group is preferred. Methyl group is especially preferred as R_{1b} in view of pharmaceutical activity.

The "halogen atom" represented by W includes chlorine, fluorine, bromine and iodine atoms. Of these, a chlorine atom is preferred.

The salt of the compound represented by the formula (Ib) includes a pharmacologically acceptable salt thereof such as an inorganic salt such as hydrochloride, hydrobromide, sulfate, nitrate, phosphate and the like, an organic acid salt such as acetate, tartrate, citrate, fumarate, maleate, toluenesulfonate, methanesulfonate and the like, a metal salt such as sodium salt, potassium salt, calcium salt, alminum salt and the like, a salt with base such as triethylamine salt, guanidine salt, ammonium salt, hydrazine salt, quinine salt, cinchonine and the like, and the like.

In addition, a hydrate and non-hydrate of the compound represented by the formula (Ib) are also encompassed in the present invention.

The compound represented by the formula (Ib) or a salt thereof has asymmetry carbons at the 3-position and 5-position, and a trans form in which the substituents at the 3-position and 5-position are directed to the opposite direction each other relative to the plane of the 7-membered ring is preferred, and a compound in which the absolute configuration of the 3-position is R configuration and the absolute configuration of the 5-position is S-configuration is specifically preferred.

As the compound represented by the formula (Ib) or a salt thereof, the following compounds are specifically preferred.
N-methanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide, N-[2-(pyrrolidin-1-yl)ethyl]-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide, N-[2-(pyrrolidin-1-yl)ethyl]-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-1-[3-acetoxy-2,2-dimethylpropyl)-7-chloro-5-(2,3-dimethoxyphenyl)-2-oxo-1,2,3,5-tetrahydro-4,1-benzoxazepin-3-yl]acetamide, N-methanesulfonyl-[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetamide, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid, N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester, N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester, 3R,5S)-7-chloro-5-(2,3-dimethoxyphenyl)-1-(3-hydroxy-2,2-dimethylpropyl)-1,2,3,5-tetrahydro-3-[1H (or 3H)-tetrazol-5-yl]methyl-4,1-benzoxazepin-2-one, (3R,5S)-7-chloro-5-(2,3-dimethoxyphenyl)-1-(3-hydroxy-2-hydroxymethyl-2-methylpropyl)-1,2,3,5-tetrahydro-3-[1H (or 3H)-tetrazol-5-yl]methyl-4,1-benzoxazepin-2-one, (3R,5S)-1-(3-acetoxy-2,2-dimethylpropyl-7-chloro-5-(2,3-dimethoxyphenyl)-1,2,3,5-tetrahydro-3-[lH (or 3H)-tetrazol-5-yl]methyl-4,1-benzoxazepin-2-one, (3R,5S)-1-(3-acetoxy-2-acetoxymethyl-2-methylpropyl)-7-chloro-5-(2,3-dimethoxyphenyl)-1,2,3,5-tetrahydro-3-[1H (or 3H)-tetrazol-5-yl]methyl-4,1-benzoxazepin-2-one N-[2-(pyrrolidin-1-yl)ethyl]-[(3R,5S)-7-chloro-5-(2,3-dimethoxyphenyl)-1-neopentyl-2-oxo-1,2,3,5-tetrahydro-4,1-benzoxazepin-3-yl]acetamide; and the like.

The compound represented by the formula (Ib) or a salt thereof can be prepared, for example, according to the method disclosed in the publications such as EPA567026, WO95/21834 (PCT application based on Japanese Patent Application No. H6-15531), EPA645377 (application based on Japanese Patent Application No. H6-229159), EPA645378 (application based on Japanese Patent Application No. H6-229160), WO9710224 and the like, or a similar mrthod thereto.

As the compound represented by the formula (I), a compound represented by the above-mentioned formula (Ic) 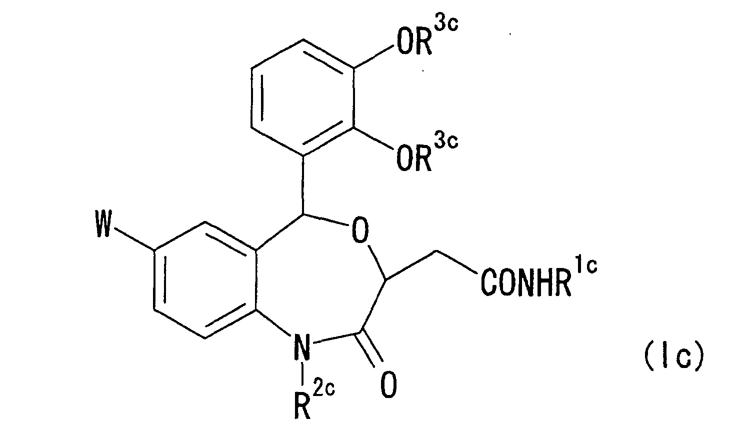 is preferred.

As the compound represented by the formula (Ic),
a compound wherein R^{1c} is a (carboxy-C₂₋₃ alkyl)-C₆₋₁₀ aryl group optionally having substituent;
a compound wherein R^{1c} is a (carboxy-C₂₋₃ alkyl)-phenyl group optionally having substituent;
a compound wherein R^{1c} is a (carboxyfuryl)-alkyl group optionally having substituent;
a compound wherein R^{2c} is a C₃₋₆ alkyl group having 1 to 3 substituents selected from a hydroxy group, an acetoxy, a propionyloxy, a tert-butoxycarbonyloxy and a palmitoyloxy;
a compound wherein R^{2c} is a 2,2-dimethylpropyl, a 3-hydroxy-2,2-dimethylpropyl or a 3-acetoxy-2,2-dimethylpropyl;
a compound wherein R^{3c} is a methyl group;
a compound wherein W is a chlorine atom;
a compound wherein the 3-position is R-configuration and the 5-position is S-configuration; and the like are preferred.

In the above-mentioned formula, R^{1c} is a 1-carboxyethyl group optionally having substituent, a carboxy-C₃₋₆ straight chain alkyl group optionally having substituent, a C₃₋₆ straight chain alkyl-sulfonyl group optionally having substituent, a (carboxy-C₅₋₇ cycloalkyl)-C₁₋₃ alkyl group optionally having substituent, or the group represented by the formula: -X¹-X²-Ar-X³-X⁴-COOH (wherein X¹ and X⁴ are each a bond or a C₁₋₄ alkylene group optionally having substituent, X² and X³ are each a bond, -O- or -S-, Ar is a divalent aromatic ring group optionally having substituent. Provided that when X¹ is a bond, X² is a bond, and when X⁴ is a bond, X³ is a bond).

The C₃₋₆ straight chain alkyl group of the carboxy-C₃₋₆ straight chain alkyl group optionally having substituent represented by R^{1c} includes n-propyl, n-butyl, n-pentyl, n-hexyl. Of these, n-propyl, n-butyl are preferred, and n-propyl is more preferred.

In the above-mentioned formula, the C₃₋₆ straight chain alkyl group of a C₃₋₆ straight chain alkyl-sulfonyl group optionally having substituent represented by R^{1c} includes n-propyl, n-butyl, n-pentyl, n-hexyl. Of these, n-propyl, n-butyl are preferred, and n-propyl is more preferred.

The C₅₋₇ cycloalkyl group of the (carboxy-C₅₋₇ cycloalkyl)-C₁₋₃ alkyl group optionally having substituent represented by R^{1c} includes cyclopentyl, cyclohexyl, cycloheptyl. Of these, cyclopentyl, cyclohexyl are preferred, and cyclohexyl are more preferred.

The C₁₋₃ alkyl group of the (carboxy-C₅₋₇ cycloalkyl)-C₁₋ ₃ alkyl group optionally having substituent represented by R^{1c} includes methyl, ethyl, n-propyl, isopropyl. Of these, methyl, ethyl are preferred, and methyl is more preferred.

In the group represented by the formula: -X¹-X²-Ar-X³-X⁴-COOH as R^{1c}, the "C₁₋₄ alkylene group" of the "C₁₋₄ alkylene group optionally having substituent" represented by X¹ and X⁴ includes for example methylene, dimethylene, trimethylene, tetramethylene and the like, and a C₁₋₃ alkylene group is preferred. Of these, a group having a straight chain is preferably used.

The "divalent aromatic ring group" of the "divalent aromatic ring group optionally having substituent" represented by Ar includes for example a divalent aromatic hydrocarbon group, a divalent aromatic heterocyclic group and the like.

Here, the divalent aromatic hydrocarbon group includes for example a group formed by eliminating one hydrogen atom from a C₆₋₁₀ aryl group (e.g., phenyl, naphthyl and the like), and phenylene is preferably used as the divalent aromatic hydrocarbon group.

The divalent aromatic heterocyclic group includes for example a group formed by eliminating one hydrogen atom from an aromatic heterocyclic group containing at least one (preferably 1 to 4, more preferably 1 or 2) of 1 to 3 (preferably 1 to 2) kinds of heteroatoms selected from an oxygen atom, a sulfur atom and nitrogen atom and the like as the ring-constituting atoms (ring atom), and the like.

Herein, the aromatic heterocyclic group includes for example a 5- or 6-membered aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like (preferably, furyl, thienyl, pyrrolyl, imidazolyl, thiazolyl, pyridyl and the like), and an 8 to 12-membered aromatic fused heterocyclic group such as benzofuranyl, isobenzofuranyl, benzo[b]thienyl, indolyl, isoindolyl, 1H-indazolyl, benzimidazolyl, benzoxazolyl, 1,2-benzoisoxazolyl, benzothiazolyl, benzopyranyl, 1,2-benzoisothiazolyl, 1H-benzotriazolyl, quinolyl, isoquinolyl, cynnolinyl, quinazolinyl, quinoxanyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenathridinyl, phenathrolinyl, indolizinyl, pyrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like (preferably, a heterocyclic ring in which the above-mentioned 5- or 6-membered aromatic monocyclic heterocyclic group is fused to a benzene ring, or a heterocyclic ring in which the same or different two heterocyclic rings of the above-mentioned 5- or 6-membered aromatic monocyclic heterocyclic group are fused, more preferably a heterocyclic ring in which the above-mentioned 5- or 6-membered aromatic monocyclic heterocyclic group is fused to a benzene ring) and the like.

The substituents of each of the "C₁₋₄ alkylene group" of the "C₁₋₄ alkylene group optionally having substituent" represented by X¹ and X⁴; and the "divalent aromatic ring group" of the "divalent aromatic ring group optionally having substituent" represented by Ar include, (i) a carboxyl group optionally esterified with a C₁₋₆ alkyl group or a C₆₋₁₀ aryl-C₁₋₄ alkyl group (e.g., methyl, ethyl, propyl, isopropyl, butyl, tert-butyl, phenyl, benzyl and the like), (ii) a phosphoric acid group optionally mono- or di-substituted with a C₁₋₆ alkyl (e.g., methyl, ethyl, n-propyl, isopropyl, n-butyl, isobutyl, n-pentyl, isopentyl, neopentyl, hexyl and the like) or a C₂₋₇ alkanoyloxy-C₁₋₆ alkyl such as acetoxymethyl, pivaloyloxymethyl group, (iii) a sulfonic acid group, (iv) a sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl and the like), (v) a hydroxy group and a sulfhydryl group optionally alkylated with a C₁₋₃ alkyl group (e.g., methyl, ethyl, propyl and the like), (vi) a carbamoyl group, (vii) a phenyl group optionally substituted with 1 to 5 substituents [for example, hydroxy group, chlorine, fluorine, aminosulfonyl group, an amino group optionally substituted with a C₁₋₃ alkyl group (e.g., methyl, ethyl, propyl and the like)] and optionally bound to via O or S, (viii) an amino group optionally mono- or di- substituted with a C₁₋₃ alkyl group (e.g., methyl, ethyl, propyl and the like), (ix) a cyclic amino group optionally substituted with 1 to 3 of a C₁₋₃ alkyl (e.g., methyl, ethyl and the like), benzyl, phenyl and the like (e.g., a 5 to 6-membered cyclic amino group optionally containing an oxygen atom, a sulfur atom as ring-constituting atoms in addition to nitrogen atom, such as a cyclic amino group derived from (by eliminating one hydrogen atom) a cyclic amine, such as piperidine, pyrrolidine, morpholine, thiomorpholine, piperazine, 4-methylpiperazine, 4-benzylpiperazine, 4-phenylpiperazine, 1,2,3,4-tetrahydroisoquinoline, phthalimide and the like), (x) a 5- or 6-membered aromatic heterocyclic group containing 1 to 4 heteroatoms selected from N, O, S, and optionally bound to via O or S (e.g., pyridyl, imidazolyl, indolyl, tetrazolyl and the like), (xi) a halogen atom (e.g., chlorine, fluorine, bromine, iodine and the like), (xii) a C₁₋₄ alkyl group (e.g., methyl, ethyl, propyl, isopropyl, butyl, t-butyl and the like), a C₁₋₄ alkoxy group (e.g., methoxy, ethoxy, propoxy, isopropoxy, butoxy, t-butoxy and the like) or a C₁₋₄ alkylthio group (e.g., methylthio, ethylthio, propylthio, isopropylthio, butylthio, t-butylthio and the like), each of which is optionally substituted with substituent selected from a C₁₋₄ alkoxy group, a C₁₋₄ alkylthio group, a carboxyl and a phenyl, (xiii) a C₅₋₇ cycloalkyl group (e.g., cyclopentyl, cyclohexyl, cycloheptyl and the like), (xiv) a C₁₋₇ alkanoyloxy (e.g., formyloxy, acetoxy, propionyloxy, butyryloxy, t-butoxycarbonyloxy, isobutyryloxy, valeryloxy, pivaloyloxy and the like). One to six, preferably one to three of such substituents may exist at the substitutable position. Alternatively, two of these substituents may be bound to form a C₃₋₆ alkylene, a C₃₋₆ alkyleneoxy, a C₃₋₆ alkylenedioxy and the like, and for example, when the adjacent two substituents on a phenyl group are bound to form a C₄ alkylene, a tetrahydronaphthalene group is formed.

Specific examples of the group represented by the formula -X¹-X²-Ar-X³-X⁴-COOH as R^{1c}, a (carboxy-heteroaryl)-C₁₋₄ alkyl group optionally having substituent [preferably, a (carboxy-furyl)-C₁₋₄ alkyl group optionally having substituent], a (carboxy-C₆₋₁₀ aryl)-C₁₋₄ alkyl group optionally having substituent, a carboxy-heteroaryl group optionally having substituent, a carboxy-C₆₋₁₀ aryl group optionally having substituent, a (carboxy-C₁₋₄ alkyl)-hetero aryl group optionally having substituent, a (carboxy-C₁₋₄ alkyl)-C₆₋₁₀ aryl group optionally having substituent [preferably, a (carboxy-C₂₋₃ alkyl)-C₆₋₁₀ aryl group], a (carboxy-C₁₋₄ alkyl)-heteroaryl-C₁₋₄ alkyl group optionally having substituent, a (carboxy-C₁₋₄ alkyl)-C₇₋₁₄ aralkyl group optionally having substituent [preferably, a (carboxy-C₁₋₃ alkyl)-C₇₋₁₄ aralkyl group optionally having substituent], a (carboxy-C₁₋₄ alkoxy)-C₆₋₁₀ aryl group optionally having substituent, a (carboxy-C₁₋₄ alkoxy)-C₆₋₁₀ aryl-C₁₋₄ alkyl group optionally having substituent, a (carboxy-C₁₋₄ alkyl)-C₆₋₁₀ aryloxy-C₁₋₄ alkyl group optionally having substituent, a (carboxy-C₆₋₁₀ aryloxy)-C₁₋₄ alkyl group optionally having substituent, a (carboxy-C₁₋₄ alkylthio)-heteroaryl group optionally having substituent and the like.

As used herein, the heteroaryl includes those exemplified as the "aromatic heterocyclic group" above, and the heteroaryl may have substituent similar to those the above-mentioned "aromatic heterocyclic group" may have. The C₆₋₁₀ aryl includes phenyl, naphthyl, azulenyl, and phenyl is preferably used. The C₆₋₁₀ aryl may have substituent similar to those the above-mentioned "aromatic heterocyclic group" may have. In the (carboxyfuryl)-C₁₋₄ alkyl group optionally having substituent represented by R¹, the alkyl group includes a C₁₋₄ straight or branched alkyl group such as methyl, ethyl, n-propyl, isopropyl, n-butyl, isobutyl, 1,1-dimethylethyl and the like, and the like. Of these, a C₁₋₄ alkyl group such as methyl, ethyl, n-propyl, isopropyl, n-butyl and the like are preferred, and methyl, ethyl, n-propyl are more preferred. The carboxyfuryl group includes for example 3-carboxy-2-furyl, 4-carboxy-2-furyl, 2-carboxy-3-furyl, 2-carboxy-5-furyl and the like. Of these, 3-carboxy-2-furyl, 4-carboxy-2-furyl are preferred, and 3-carboxy-2-furyl is more preferred.

The C₂₋₃ alkyl of the (carboxy-C₂₋₃ alkyl)-C₆₋₁₀ aryl group optionally having substituent represented by R^{1c} includes ethyl, n-propyl and isopropyl, and ethyl and n-propyl are preferred. The C₆₋₁₀ aryl group includes phenyl, naphthyl and azulenyl, and phenyl is preferred.

The C₁₋₃ alkyl group of the (carboxy-C₁₋₃ alkyl)-C₇₋₁₄ aralkyl group optionally having substituent represented by R^{1c} includes methyl, ethyl, n-propyl and isopropyl, and methyl, ethyl are preferred, and ethyl is specifically preferred. The C₇₋₁₄ aralkyl group (C₆₋₁₀ aryl-C₁₋₄ alkyl group) includes phenylmethyl, 1-phenylethyl, 2-phenylethyl, 3-phenylpropyl, 2-phenylpropyl, 4-phenylbutyl, (1-naphthyl)methyl, (2-naphthyl)methyl, 1-(1-naphthyl)ethyl, 1-(2-naphthyl)ethyl, 3-(1-naphthyl)propyl, 3-(1-naphthyl)propyl, 4-(1-naphthyl)butyl and 4-(2-naphthyl)butyl, and phenylmethyl, 1-phenylethyl, 3-phenylpropyl, (1-naphthyl)methyl, (2-naphthyl)methyl, (1-naphthyl)ethyl, (2-naphthyl)ethyl are preferred, and phenylmethyl, 2-phenylethyl are specifically preferred.

The substituents include, when the each group represented by R^{1c} has substituent, the substituents similar to the "divalent aromatic ring group" of the "divalent aromatic ring group optionally having substituent" represented by Ar may have, and 1 to 6, preferably 1 to 3 of such substituents may exist at the substitutable positions. In addition, in each of the groups represented by R¹, the carboxyl portion is preferably unsubstituted, but any portions except the carboxyl may have substitutable substituent at the substitutable position.

As R^{1c}, a 3-carboxypropyl group; a 1-carboxyethyl group; a C₃₋₆ straight chain alkyl-sulfonyl group, a (carboxy-C₅₋₇ cycloalkyl)-C₁₋₃ alkyl group, a (carboxyfuryl)-alkyl group, a carboxy-C₆₋₁₀ aryl group, a (carboxy-C₁₋₄ alkyl)-C₆₋₁₀ aryl group [preferably, a (carboxy-C₂₋₃ alkyl)-C₆₋₁₀ aryl group], a (carboxy-C₁₋₃ alkyl)-C₇₋₁₄ aralkyl group, each of which optionally has substituent; and the like are preferred, and a (carboxy-C₁₋₄ alkyl)-C₆₋₁₀ aryl group optionally having substituent are preferred, and a (carboxy-C₂₋₃ alkyl)-C₆₋₁₀ aryl group optionally having substituent is more preferred. Specifically, a (carboxy-C₂₋₃ alkyl)-phenyl group optionally having substituent is preferred.

The C₃₋₆ alkyl group of the C₃₋₆ alkyl group optionally substituted with an alkanoyloxy group or a hydroxy group represented by R^{2c} include for example n-propyl, isopropyl, 1,1-dimethylethyl, n-butyl, isobutyl, n-pentyl, 2,2-dimethylpropyl, isopentyl, n-hexyl, isohexyl and the like. Of these, isopropyl, 1,1-dimethylethyl, n-butyl, isobutyl, 2,2-dimethylpropyl, isohexyl are preferred, and 2,2-dimethylpropyl is specifically preferred.

The alkanoyloxy group of the C₃₋₆ alkyl optionally substituted with an alkanoyloxy group or a hydroxy group represented by R^{2c} include a C₁₋₂₀ alkanoyloxy group (preferably, a C₁₋₇ alkanoyloxy group and the like) such as formyloxy, acetoxy, propionyloxy, butyryloxy, tert-butoxycarbonyloxy, isobutyryloxy, valeryloxy, pivaloyloxy, lauryloxy, palmitoyloxy, stearoyloxy and the like. Of these, acetoxy, propionyloxy, tert-butoxycarbonyloxy, palmitoyloxy are preferred, and acetoxy is specifically preferred. The substitutable position may be substituted with 1 to 3 of the alkanoyloxy group or hydroxy group.

Preferred examples of the C₃₋₆ alkyl optionally substituted with an alkanoyloxy group or a hydroxy group represented by R^{2c} include 2,2-dimethylpropyl, 3-hydroxy-2,2-dimethylpropyl, 3-hydroxy-2-hydroxymethyl-2-methylpropyl, 3-acetoxy-2,2-dimethylpropyl, 3-acetoxy-2-hydroxymethyl-2-methyl-propyl and 3-acetoxy-2-acetoxymethyl-2-methylpropyl and the like. Of these, 2,2-dimethylpropyl, 3-hydroxy-2,2-dimethylpropyl, 3-acetoxy-2,2-dimethylpropyl are specifically preferred.

Furthermore, as R², a C₃₋₆ alkyl group having an alkanoyloxy group and/or a hydroxy group is preferred.

The lower alkyl group represented by R^{3c} includes a C₁₋₆ alkyl group such as methyl, ethyl, n-propyl, isopropyl, n-butyl, tert-butyl, pentyl, hexyl and the like. Specifically, a C₁₋₃ alkyl group is preferred. Methyl group is especially preferred as R^{3c} in view of pharmaceutical activity.

The halogen atom represented by W includes chlorine, fluorine, bromine, iodine atoms. Of these, a chlorine atom is preferred.

Both a free form and a pharmacologically acceptable salt of the compound represented by the formula (Ic) are encompassed in the present invention. Such salt may form, when the compound of the formula (Ic) has an acidic group such as a carboxyl group and the like, a salt with an inorganic base (e.g., an alkaline metal such as sodium, potassium and the like, an alkaline earth metal such as calcium, magnesium and the like, a transition metal such as zinc, iron, copper and the like) or an organic base (e.g., an organic amine such as trimethylamine, triethylamine, pyridine, picoline, ethanolamine, diethanolamine, triethanolamine, dicyclohexylamine, N,N'-dibenzylethylenediamine and the like, a basic amino acid such as arginine, lysine, ornithine and the like) and the like.

When the compound of the formula (Ic) of the present invention has a basic group such as an amino group and the like, it may form a salt with an inorganic acid or an organic acid (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like), or an acidic amino acid such as aspartic acid, glutamic acid and the like, and the like.

The compound represented by the formula (Ic) or a salt thereof has asymmetry carbons at the 3-position and the 5-position, respectively, and it may be a mixture of steric isomers, which may be resolved by known means. A trans form, which is an isomer in which the substituents at the 3-position and 5-position are directed to the opposite direction each other relative to the plane of the 7-membered ring, is preferred, and specifically, a form in which the absolute configuration of the 3-position is R configuration and the absolute configuration of the 5-position is S configuration is preferred. Alternatively, the compound may be a racemate or an optically active form. The optically active compound may be resolved from racemate according to a known mean for optical resolution.

As the compound represented by the formula (Ic) or a salt thereof of the present invention, the following compounds and the like are preferred. N-propanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide or a salt thereof, (2R)-2-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminopropionic acid or a salt thereof, 3-[3-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminophenyl]propionic acid or a salt thereof, 4-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminobutanoic acid or a salt thereof, trans-4-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]-aminomethyl-1-cyclohexanecarboxylic acid or a salt thereof, trans-4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]-aminomethyl-1-cyclohexanecarboxylic acid or a salt thereof, 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-fluorophenyl]propionic acid or a salt thereof, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methylphenyl]propionic acid or a salt thereof, 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-methylphenyl]propionic acid or a salt thereof, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl]phenyl]propionic acid or a salt thereof, 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl]phenyl]propionic acid or a salt thereof, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]propionic acid or a salt thereof, 2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid or a salt thereof, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazen-3-yl]acetyl]amino]-4-fluorophenyl]propionic acid or a salt thereof, 3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid or a salt thereof, 4-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]butanoic acid or a salt thereof, 5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]pentanoic acid or a salt thereof, 5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]pentanoic acid or a salt thereof, and the like.

The above-mentioned compounds represented by the formula (Ic) or a salt thereof can be prepared by methods disclosed in, publications such as EPA567026, WO95/21834 (PCT application based on Japanese Patent Application No. H6-15531), EPA645377 (application based on Japanese Patent Application No. H6-229159), EPA645378 (application based on Japanese Patent Application No. H6-229160) and the like, the description of Japanese Patent Application No. 2000-190253 (PCT/JP01/05347) and the like, or a similar method thereto.

As the raw material compounds of the compound represented by the formula (I) of the present invention, the salts similar to those as mentioned above are used, and are not specifically limited so long they do not adversely affect the reactions.

The high-density lipoprotein-cholesterol level elevating agent of the present invention has superior high-density lipoprotein-cholesterol level elevation activity and is low toxic. Therefore, these compounds and salts thereof may be used safely as, for example, an agent for prevention or treatment of primary hypoalphalipoproteinemia, an agent for prevention or treatment of Tangier disease and the like, as well as an agent for prevention or treatment of heart infarction, an agent for prevention or treatment of atherosclerotic disease, an agent for prevention or treatment of hyperlipemia, an agent for prevention or treatment of familial hypercholesterolemia, an agent for prevention or treatment of diabetes mellitus, an agent for prevention or treatment of diabetic complication and the like, in a mammal (e.g., mouse, rat, hamster, rabbit, cat, dog, cattle, horse, sheep, monkey, human and the like).

While in the high-density lipoprotein-cholesterol level elevating agent and an agent for preventing or treating familial hypercholesterolemia of the present invention, a compound having squalene synthase inhibitory activity or a salt thereof, or a prodrug thereof (an SSI compound or a prodrug thereof) as an active ingredient may be administrated as original powder, it is generally administered as a form of a preparation prepared according to a general method, using a general suitable carrier for preparation in a suitable amount, and such carrier includes for example an excipient (e.g., calcium carbonate, kaolin, sodium hydrogencarbonate, lactose, starches, crystalline cellulose, talc, granulated sugar, porous substance and the like), a binder (e.g., dextrin, rubbers, alcoholated starch, gellatin, hydroxypropyl cellulose, hydroxypropyl methylcellulose, pullulan and the like), a disintegrator (e.g., carboxymethylcellulose calcium, crosscarmelose sodium, crosspovidone, hydroxypropyl cellulose, partial pregelatinized starch and the like), a lubricant (e.g., magnesium stearate, calcium stearate, talc, starch, sodium benzoate and the like), a coloring agent (e.g., tar dye, caramel, ferric oxide, titanium oxide, riboflavins and the like), a flavoring agent (e.g., sweetening agents, flavoring agent and the like), stabilizer (e.g., sodium sulfite and the like) and a preservative (e.g., parabens, sorbic acid and the like) and the like. The agent of the present invention comprising the above-mentioned preparation suitably comprises an SSI compound or a prodrug thereof in an effective amount to prevent and treat disease. The content of the SSI compound or a prodrug thereof in the preparation of the present invention is generally 0.1 to 100% by weight relative to the whole preparation. Alternatively, the preparation used in the present invention may comprise other medicament ingredients besides an SSI compound or a prodrug thereof, and these ingredients are not specifically limited so long the object of the present invention is achieved, and can be used in a suitable ratio of incorporation. As the specific examples of dosage form, for example tablet (including sugar-coated tablet, film-coated tablet), pill, capsule, granulate, grain, powder, syrup, emulsifier, suspension, injection, sustained-release injection, inhalation, ointment and the like, are used. These preparations are prepared according to a general method (e.g. methods described in The Pharmacopoea of Japan, and the like).

Specifically, a tablet may be prepared by a method comprising granulating an SSI compound or a prodrug thereof as it is or as a homogenous mixture with excipient, binder, disintegrator or other suitable additive by a suitable procedure, adding lubricant and the like thereto and compression-molding the mixture to give a tablet, or a method comprising directly compression-molding an SSI compound or a prodrug thereof as it is or as a homogenous mixture with excipient, binder, disintegrator or other suitable additive to give a tablet, or a method comprising compression-molding a granular that has been previously prepared, as it is, or as a homogenous mixture with a suitable additive to give a tablet. Furthermore, the present agent may optionally comprise a coloring agent, a flavoring agent and the like. Moreover, the present agent may be coated with a suitable coating agent. The injection may be produced by a production method comprising dissolving, suspending or emulsifying an SSI compound or a prodrug thereof in a predetermined amount, in water for injection, saline, Ringer's solution and the like (in the case of an aqueous solvent), or a vegetable oil and the like (in the case of a non-aqueous solvent) to adjust the amount to a certain amount, or a method comprising sealing an SSI compound or a prodrug thereof in a container for injection in a predetermined amount.

As a carrier for oral preparation, substances used in the art of drug preparation such as starch, mannitol, crystalline cellulose, carboxymethylcellulose sodium and the like are used. As a carrier for injection, for example, distilled water, saline, glucose solution, infusion solution and the like are used. Additionally, other additives used for general preparation may be suitably added.

Alternatively, the preparation of the present invention may be used as a sustained-release preparation. The sustained-release preparation of the present invention can be administered as microcapsules as it is by preparing, for example, by drying-in-water method (o/w method, w/o/w method and the like), phase separation method, spray drying or a similar manner thereto (e.g., microsphere microcapsules, microparticles and the like), or as various formulations by preparing the microcapsule or a pharmaceutical composition in sphere form, needle form, pellet form, film form or cream form as raw materials into various dosage forms. The dosage form includes for example a parenteral agent (e.g., an injection or an implanted agent for intramuscular, subcutaneous, organ and the like; an intramucosal agent for nasal cavity, rectum, uterus and the like, and the like), an oral agent (e.g., hard capsule, soft capsule, granulate, powder, suspension and the like) and the like.

When the sustained-release preparation of the present invention is an injection, the sustained-release injection is prepared by dispersing the microcapsule in a dispersing agent (e.g., a surfactant such as Tween 80, HCO-60 and the like; polysaccharides such as carboxymethylcellulose, sodium arginate, sodium hyaluronate and the like; protamine sulfate, polyethyleneglycol and the like), a preservative (e.g., methylparaben, propylparaben and the like), an isotonic agent (e.g., sodium chloride, mannitol, sorbitol, glucose and the like), a local anesthetic (e.g., xylocaine hydrochloride, chlorobutanol and the like) and the like to give an aqueous suspension, or dispersing the microcapsule in a vegetable oil (e.g., sesame oil, corn oil and the like) or a mixture thereof with a phospholipid (e.g., lecithin and the like) or a middle chain fatty acid triglyceride (e.g., Miglyol 812 and the like) to give an oily suspension.

When the sustained-release preparation of the present invention is a microcapsule, its mean particle diameter is about 0.1 to about 300 µm, preferably about 1 to about 150 µm, more preferably about 2 to about 100 µm.

While the means for sterilize the microcapsules include a method comprising sterilizing the whole steps of preparation, a method comprising sterilizing using gamma ray, a method comprising adding an antiseptic and the like, the method is not specifically limited.

The high-density lipoprotein-cholesterol level elevating agent of the present invention is low toxic and useful as a medicament, and has superior high density lipoprotein-cholesterol level elevating action. Therefore, the agent of the present invention is useful as an agent for prevention or treatment of diseases based on this pharmacological action.

Namely, the agent can be used for prevention or treatment of arterial sclerosis, hyperlipemia, diabetes mellitus, diabetic complication, diabetic nephropathy, diabetic neuropathy, diabetic retinopathy, allorhythmic pulse, periphery vascular disease, thrombosis, pancreatic disease, ischemic heart disease, cerebral ischemia, secondary disease of heart infarction, cardiac valvular disease, Alzheimer's disease and the like. In addition, the agent is suitable for prevention or treatment of familial hypercholesterolemia, primary hypoalphalipoproteinemia, Tangier disease, and ischemic heart disease that frequently occurs in a patient after menopause, who is suffering from diabetes mellitus.

The high-density lipoprotein-cholesterol level elevating agent of the present invention is more useful for prevention or treatment of primary hypoalphalipoproteinemia, Tangier disease and the like than an agent that has LDL lowering action but shows no HDL elevating action, since the LDL lowering action solely provides no treatment effect to these diseases. The final object of a therapeutic agent for hyperlipemia is to prevent the onset of lethal diseases such as heart infarction and the like. While even a drug that has LDL lowering action but has no HDL elevating effect shows prevention effect for the onset of heart infarction and the like on some level, the high-density lipoprotein-cholesterol level elevating agent can prevent the onset of heart infarction and the like more strongly. Furthermore, the agent is also effective for patients, diseases or symptoms (e.g., intractable hyperlipemia and the like) in which any treatment effect by a drug that has LDL lowering action but has no HDL elevating action has not been observed. Moreover, the agent can suppress the incidence of lethal diseases such as heart infarction and the like and improve the treatment effect in a human having a normal level of blood serum lipid.

Furthermore, the agent is specifically suitable for the treatment and prevention of hyperlipemia, specifically hypertriglyceridemia, hyperlipoproteinemia and hypercholesterolemia and atherosclerosis vascular lesion arising therefrom, as well as the secondary diseases thereof such as coronary artery disease, cerebral ischemia, aneurysm, cerebral arteriosclerosis, periphery arterial sclerosis, claudicatio intermittens, gangraena and the like.

Moreover, as a further notable example of application of the high-density lipoprotein-cholesterol level elevating agent of the present invention, prevention and treatment of Alzheimer's disease is exemplified. It has been known that increase of blood choresterol is a risk factor of Alzheimer's disease. An SSI compound such as the compound of the formula (I) or a salt thereof, or a prodrug thereof and the like can be used for the prevention and treatment of Alzheimer's disease, due to their superior high density lipoprotein-cholesterol level elevating action and lipid lowering action, and when the SSI compound is used for such purpose, it can be administered solely or in combination with the drugs exemplified below. In this case, the possible combination includes a combination with an acetylcholinesterase inhibitor (e.g., Aricept, Excelon and the like), an amyloid β-production and secretion inhibitor (e.g., a γ or β secretase inhibitor such as JT-52, LY-374973 and the like, or SIB-1848 and the like), an amyloid β coagulation inhibitor (e.g., PTI-00703, BETABLOC (AN-1792) and the like) and the like.

The compound of the formula (I) or a salt thereof, a prodrug thereof of the present invention (hereinafter sometimes referred to merely as the compound of the formula (I) or the compound (I), including a salt thereof and a prodrug thereof) has low toxicity and high density lipoprotein-cholesterol level elevating action. Furthermore, since the compound has squalene synthase inhibitory action, triglyceride lowering action as well as superior lipid lowering action, it is useful as a safe medicament for the prevention or treatment of hyperlipemia such as hypercholesterolemia, hypertriglyceridemia and the like, in a mammal (e.g., mouse, rat, rabbit, dog, cat, cattle, pig, monkey, human and the like). Moreover, the compound is useful as a safe medicament for the the prevention or treatment of kidney disease such as nephritis, nephropathy and the like, arterial sclerosis, ischemic disease, heart infarction, stenocardia, aneurysm, cerebral arteriosclerosis, periphery arterial sclerosis, thrombosis, hypertension, osteoporosis, diabetes mellitus (e.g., insulin resistance-dependent type and the like), pancreatic disease, re-stricture after percutaneous coronary angioplasty (PTCA).

Hereinafter the utility of the present invention is explained in detail.

The compound of the formula (I) has superior triglyceride lowering action and cholesterol lowering action as well as biological characteristics thereof, and is specifically suitable for the treatment or prevention of hyperlipemia, specifically hypertriglyceridemia, hyperlipoproteinemia and hypercholesterolemia, and atherosclerotic vascular lesion arising therefrom, as well as the secondary disease thereof such as coronary artery disease, cerebral ischemia, cerebral stroke, claudicatio intermittens, gangraena and the like.

During the treatment of these diseases, the compound of the formula (I) may be used solely for the treatment, or may be used in combination with the other pharmaceutical ingredients such as a lipid lowering agent or a cholesterol lowering agent and the like (administered simultaneously or administered at intervals), and in these cases, the compound is preferably administered as an oral preparation, or it may be optionally administered in the form of suppository as a rectal preparation. In the above cases, the possible ingredients for the combination use include, for example, a PPAR_{α} agonist such as fibrates [e.g., Clofibrate, Benzafibrate, Gemfibrozil, Fenofibrate, Wy-1463, GW9578 and the like] and the like, nicotinic acid, a derivative thereof and an analogue thereof [e.g., Acipimox and Probucol], a bile acid-binding resin [e.g., Cholestyramine, Colestipol and the like], a compound that suppresses absorption of cholesterol [e.g., Sitosterol, Neomycin, β-lactam derivative and the like], a compound that inhibits biosynthesis of cholesterol [e.g., an HMG-CoA reductase inhibitor such as Lovastatin, Simvastatin, Pravastatin, Atorvastatin, ZD-4522, Itavastatin and the like], a squalene epoxydase inhibitor [e.g., NB-598 and an analogue compound thereof and the like].

Other possible ingredients for the combination use include an oxide squalene-lanosterol cyclase inhibitor (e.g., a decalin derivative, an azadecalin derivative and an indan derivative and the like), MTP (a microsomal triglyceride transfer protein inhibitor (Implitapide and the like) and the like.

In addition, the compound of the formula (I) is suitable for the treatment of diseases relating to hyperchylomicronemia such as acute pancreatitis and the like. The mechanism of the onset of pancreatitis is said to occur due to microembolization in the pancreatic capillary blood vessel by chylomicron, or due to strong stimulation of the focus by free fatty acid that has been produced by digestion of triglyceride with pancreatic lipase due to hyperchylomicronemia. Therefore, since the compound of the formula (I) of the present invention has triglyceride lowering action, it can be used for the treatment of pancreatitis, and it can be used solely or in combination with a known method for treatment pancreatitis, for the treatment of pancreatitis. For the treatment of this disease, the compound (I) of the present invention can be administered orally or locally, and it can be used solely or in combination with a known active compound. In this case, the possible ingredients for the combination include aprotinin (Trasylol), gabexate mesilate (FOY), nafamostat mesilate (Fusan), citicoline (Nicoline), urinastatin (Miraclid) for antifermentative treatment, and the like. For the purpose of alleviation of pain, an anticholinergic agent, a non-narcotic analgesic, a narcotic are also used.

One of the further notable examples of the application of the compound of the formula (I) is application for secondary hyperlipemia. The disease includes diabetes mellitus, hypothyreosis, nephrotic syndrome or chronic renal failure and the like, and hyperlipemia onsets subsequent to these diseases. In many cases, hyperlipemia aggravates these diseases, namely, forms a vicious circle. In view of lipid lowering action, the compound of the formula (I) is also suitable for the treatment or prevention of development of these diseases, and in this case, it can be administered solely or in combination with the medicaments listed below, and preferably by oral administration.

A therapeutic agent for diabetes mellitus: Kinedak, Avandia, Penfil, Humalin, Euglucon, Glimicron, Daonil, Novolin, Monotard, insulins, Glucobay, Dimelin, Rastinon, bacilcon, Deamelin S, iszilins;
a therapeutic agent for hypothyreosis: dried thyroid (Thireoid), levothyroxine sodium (Thyradin S) , liotyronin sodium (Thyronine, Thyromine);
a therapeutic agent for nephrotic syndrome: prednisolone (Predonine), prednisolone sodium succinate (Predonine), methylprednisolone sodium succinate (Solu-Medrol), betamethasone (Lynderon);
an agent for anticoagulant therapy: dipyridamol (Persantin) , dilazep hydrochloride (Comelian) and the like;
an therapeutic agent for chronic renal failure: a diuretic [e.g., furosemide (Lasix), bumetanide (Lunetoron), azosemide (Diart) ], an antihypertensive agent (e.g., ACE inhibitor , (enalapril maleate (Renivace)) and a Ca antagonist (maninhiron), α receptor blocker and the like.

Since hyperlipemia aggravates arterial sclerosis and causes hypertension, the compound of the formula (I) is suitable for the treatment or prevention of hypertension, and in this case, the compound of the formula (I) can be administered solely or in combination with the medicaments listed below. In this case, the possible combination is, for example, combination with an angiotensin-II antagonist [e.g., losartan potassium (Nu-Lotan), candesaltan lexetil (Blopress) and the like], an ACE inhibitor [e.g., enalapril maleate (Renivace), lisinopril (Zestril, Longes), delapril hydrochloride (Adecut), captopril and the like], a calcium antagonist [e.g., amlodipine tosylate (Amlodin, Norvasc), manidipine hydrochloride (Calslot) and the like], a hypotensive diuretic, an α receptor blocker, a β receptor blocker and the like.

The further notable indication of the compound of the formula (I) is osteoporosis accompanied by increase of blood cholesterol. Due to superior lipid lowering action of the compound of the formula (I), the compound can be used for treatment or prevention of osteoporosis accompanied by increase of blood cholesterol, and in this case, the compound of the formula (I) can be administered solely or in combination with the medicaments listed below. In this case, the possible combination is, for example, combination with a sex hormone and associated drugs [e.g., an estrogen preparation, ipriflavone (Osten), raloxifene, osteron, tibolone and the like], a bone resorption inhibitor such as calcitonins, vitamin D preparations [e.g., alfacalcidol, calcitriol and the like], bisphosphonates (e.g., etidronate, clodronate and the like) and the like, a bone formation-accelerating agent such as fluorine compound, PTH and the like.

Further possible application of the compound of the formula (I) of the present invention is suppression of thrombogenesis. Since blood triglyceride level and Factor VII that involves in blood clotting have a positive correlation, and triglyceride is decreased and clotting is supperessed by intaking ω-3 fatty acid, hypertriglyceridemia accerelates thrombogenesis. Furthermore, since the VLDL of a hyperlipemia patient increased the secretion of the plasminogen activator inhibitor from a vascular endothelial cell more strongely than that of a person having normal blood lipid, it is concerned that triglyceride (hereinafter referred to as TG) decreases the ability for fibrinolysis. Therefore, in view of the TG lowering action, the compound of the formula (I) is suitable for the prevention or treatment of thrombogenesis. In this case, the compound can be used solely or in combination with the known therapeutic agent listed below, preferably for oral administration. An agent for prevention or treatment of thrombogenesis: an agent for inhibiting blood clotting [e.g., heparin sodium, heparin calcium, warfarin calcium (Warfarin)], a thrombolytic agent [e.g., urokinase], an antiplatelet agent [e.g., aspirin, sulfin pyrazolo (Anturan), dipyridamol (Persantin), ticlopidine (Panaldine), cilostazol (Pletal)].

The above-mentioned known SSI compounds may be also used for the prevention or treatment of diseases similar to those for the compound of the formula (I), and may be used in combination with concomitant drugs similar to those for the compound of the formula (I).

In addition, since the high-density lipoprotein-cholesterol level elevating agent of the present invention has blood glucose lowering action and shows blood glucose lowering effect in a rat suffering from endomorph diabetes mellitus, the compound improves insulin resistance. The agent is, in view of its biological characteristics, especially suitable for the prevention or treatment of hyperglycemia and the secondary disease arising therefrom such as complications observed in diabetic nephropathy and renal failure, cardiovascular disease diseases such as anaemia, metabolic bone disorder, vomition, nausea, asitia, diarrhea and the like, nervous symptoms such as diphtheritic neuropathy and the like, diabetic neuropathy, diabetic retinopathy, diabetic vascular disorder, as well as insulin resistance and diseases arising therefrom such as hypertension, impaired glucose tolerance and the secondary disease such as cardiac disease, cerebral ischemia, claudicatio intermittens, gangraena and the like.

In the treatment of these diseases, the high-density lipoprotein-cholesterol level elevating agent of the present invention can be used solely for the prevention or treatment, or may be used in combination with other blood glucose lowering agents or antihypertensive agents. In this case, the compound is preferably administered as an oral preparation. Alternatively, if required, the agent may be administered as a rectal preparation, in a form of suppository. In this case, the ingredients that can be used in combination include for example (1) an insulin preparation (e.g., human insulin and the like), (2) a sulfonylurea agent (e.g., glibenclamide, gliclazide and the like), (3) an α-glucosidase inhibitor (e.g., voglibose, acarbose and the like), (4) an insulin sensitizer (e.g., Pioglytazone, troglytazone and the like), (5) an aldose reductase inhibitor (e.g., Eparestat, Tolrestat and the like), a glycation inhibitor (e.g., aminoguanidine and the like) and the like.

A combination with a therapeutic agent for gynaecologic disease (a therapeutic agent for climacteric disorder (conjugated estrogen, estradiol, testosterone enanthate, estradiol valeate and the like), a therapeutic agent for mammary cancer (tamoxifen citrate and the like), a therapeutic agent for endometriosis and hysteromyoma (leuproline acetate, danazole and the like) and the like, or a combination with these drugs and a therapeutic agent for diabetes mellitus, is also possible.

Furthermore, a combination with an antihypertensive agent is possible, and the agent includes for example (1) a diuretic (e.g., furosemide, spironolactone and the like), (2) an antiadrenergic (e.g., atenorol and the like), (3) an angiotensin II antagonist (e.g., rosartan, candesaltan and the like), (4) an angiotensin I converting enzyme inhibitor (e.g., enalapril maleate, delapril hydrochloride and the like), (5) a calcium antagonist (e.g., nifedipine, hydrochloric acid manidipin and the like) and the like.

While the dose of the preparation of the present invention varies depending on administration route, symptom, age or body weight of patient and the like, for example, when the preparation is administered orally to an adult patient as a therapeutic agent for arterial sclerosis, a blood glucose lowering agent, a therapeutic agent for diabetic complication, a therapeutic agent for primary hypoalphalipoproteinemia, a therapeutic agent for Tangier disease, a therapeutic agent for familial hypercholesterolemia and the like, the compound having squalene synthase inhibitory effect or a salt thereof, or a prodrug thereof is desirably administered 0.2 to 50 mg/day, preferably 1.5 to 30 mg/day in a portion or portions per a day. The administration route may be either oral or parenteral.

Furthermore, while the dose of the sustained-release preparation of the present invention varies depending on the duration of release besides the administration route, symptom, age or body weight of patient and the like, the amount is not specifically limited so long the effective concentration of the active ingredient is sustained in the body, and the frequency of administration may be suitably selected from, depending on the circumstance, once a day to three days, or once a week to three months and the like.

The present invention is further explained in the following Examples in more detail. These examples are merely for illustration and should not be considered to limit the present invention, and can be varied within the scope that does not deviate from the scope of the present invention.

Hereinafter an experimental result that shows the pharmacological effect of the preparation of the present invention is described.

### Test Example 1

Male common marmosets (300-500 g) were bred while feeding normal feed (CMS-1, CLEA Japan, Inc.) and water freely. The body weight was measured. Blood was taken from the femoral vein, and the total cholesterol (TC), HDL-cholesterol (HDL-C) and triglyceride (TG) in blood plasma were measured using Wako Pure Chemical kit (manufactured by Wako Pure Chemicals, Inc.) and an autoanalyzer (Hitachi 7070). The animals were devided into groups, each of which consisted of five animals. The subject compound, as a suspension in 0.5% methylcellulose, was forced oral administration to the animals once a day and for four days. On the morning of the fifth day, the body weight was measured. Blood was then taken from the femoral vein, and the total cholesterol, triglyceride and HDL-cholesterol in blood plasma were measured according to the above-mentioned method. The non-HDL-C was calculated by subtracting the HDL-cholesterol from the total cholesterol.

The results are shown in Table 1.

**Table 1**

| Subject compound: N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid | | | | |
|---|---|---|---|---|
| | dose (mg/kg/day) | Non-HDL-C (%-change) | HDL-C (%-change) | TG (%-change) |
| control group | 0 | 3.4±4 | 2.4±4 | 2.9±8 |
| subject compound-administered group | 100 | -40.4±4^{§} | 16.2±10 | -37.6±17 |
| The values are represented by Means±SEM (N=5). | | | | |
| §: Significant at P<0.01 relative to a control group (Student's t-test) | | | | |

### Test Example 2

WHHL (Watanabe heritable hyperlipidemic) rabbits (2 to 3 months-old, four male, eight female), LDL receptor-deficient animal were used. The body weight was measured. Blood was then taken from the ear central artery, and the total cholesterol (TC) and triglyceride (TG) in blood plasma were measured using Wako Pure Chemical kit (manufactured by Wako Pure Chemicals, Inc.) and an autoanalyzer (Hitachi 7070). The animals were diveded into two groups, each of which consisted of six animals. The subject compound was mixed with a basic feedstuff (RC-4, Oriental Yeast, Co., Ltd) in an amouut of 0.2%, and the mixed feedstuff was fed to the animals in an amount of 120 g per a day (corresponds to the amount of 100 mg/kg/day). To the control group was fed basic feedstuff in an amount of 120 g per a day. The blood lipid levels at the starting of administration and after finishing the administration for 14 days are shown in Table 2 and Table 3, respectively.

**Table 2**

| Subject compound: N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid | | | | |
|---|---|---|---|---|
| | TC (mg/dL) | | | |
| | dose | initial | end | % of initial |
| control group | - | 812 ± 41 | 833 ± 47 | 103 ± 3 |
| subject compound | 0.2% | 838 ± 45 | 715 ± 36 | 85 ± 2** |

**Table 3**

| Subject compound: N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid | | | | |
|---|---|---|---|---|
| | TG (mg/dL) | | | |
| | dose | initial | end | % of initial |
| control group | - | 264 ± 19 | 209 ± 19 | 79 ± 5 |
| subject compound | 0.2% | 272 ± 24 | 107 ± 10 | 40 ± 3** |

In the tables, the values are represented by Means±SEM (n=6). ** : Significant at P<0.01 relative to a control group (Student's t-test)

### Preparation Example

According to the following composition, a mixture consisting of compound A (175 g), D-mannitol (175 g), corn starch (118.65 g) and crosscarmelose sodium (105 g) is sufficiently mixed using a vertical granulator (FM-VG-10 type, manufactured by Powrex Corporation), and kneaded with an aqueous solution in which hydroxypropylcellulose (19.25 g) has been dissolved (condition for kneeding: 400 rpm, 10 min). The white-colored kneaded substance is dried using a fluidized drier (FD-3S, manufactured by Powrex Corporation) under the blow temperature of 60°C for 30 min, and granulated by, using a power mill (P-3 type, manufactured by Showa Chemical Machinery Co., Ltd.) and sieving with a 1.5 mmφ punching screen. The granular (525.14 g), crosscarmelose sodium (31 g) and magnesium stearate (1.86 g) are added and mixed using a mixer (TM-15 type, manufactured by Showa Chemical Machinery Co., Ltd.) for 5 min to give granular for tablet. The granular is formed, using a tablet forming machine (Correct 19K, manufactured by Kikusui Seisakusho Ltd.) using a 8.0 mmφ angular plane punch (180 mg, pressure 0.7 ton/cm²) to give 2,350 tablets.

### Compound A: N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid

| | |
|---|---|
| Compound A | 50 mg |
| D-Mannitol | 50 mg |
| Corn starch | 33.9 mg |
| Crosscarmelose sodium | 40 mg |
| Hydroxypropylcellulose | 5.5 mg |
| Magnesium stearate | 0.6 mg |
| Total | 180.0 mg (per a tablet) |

The obtained 50 mg tablet is orally administered once a day in the evening.

### Industrial Applicability

As the preparation of the present invention has superior high density lipoprotein-cholesterol level elevation activity, it can be used safely and advantageously as an agent for prevention or treatment of primary hypoalphalipoproteinemia, an agent for prevention or treatment of Tangier disease and the like, as well as an agent for prevention or treatment of atherosclerotic disease, an agent for prevention or treatment of hyperlipemia, an agent for prevention or treatment of familial hypercholesterolemia, an agent for prevention or treatment of diabetes mellitus, an agent for prevention or treatment of diabetic complication and the like.

## Claims

1. A high-density lipoprotein-cholesterol level elevating agent comprising a compound having squalene synthase inhibitory activity or a salt thereof, or a prodrug thereof.

2. The agent according to claim 1, which is an agent for preventing or treating primary hypoalphalipoproteinemia.

3. The agent according to claim 1, which is an agent for preventing or treating Tangier disease.

4. The agent according to claim 1, wherein the compound having squalene synthase inhibitory activity is a compound represented by the formula: 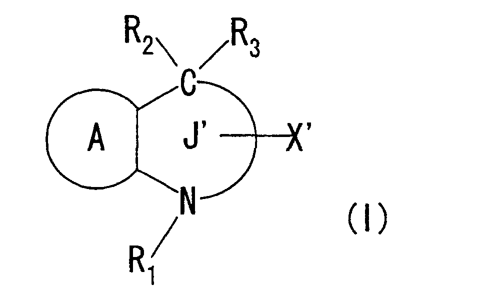 wherein R₁ is a hydrogen or an optionally substituted hydrocarbon group, R₂ and R₃ are the same or different and each is a hydrogen, an optionally substituted hydrocarbon group or an optionally substituted heterocyclic group, X' is a substituent constituted by an optionally esterified carboxyl group, an optionally substituted carbamoyl group, an optionally substituted hydroxy group, an optionally substituted amino group or an optionally substituted heterocyclic residue having a hydrogen atom that may be deprotonated, ring A is an optionally substituted benzene ring or an optionally substituted heterocyclic ring, ring J' is a 7- or 8-membered heterocyclic ring comprising three heteroatoms or less as the ring-constituting atoms, ring J' may have additional substituents besides R₁, R₂, R₃ and X',or a salt thereof.

5. The agent according to claim 4, wherein R₁ is an optionally substituted aliphatic non-cyclic hydrocarbon group.

6. The agent according to claim 5, wherein the aliphatic non-cyclic hydrocarbon group is a branched alkyl group.

7. The agent according to claim 4, wherein R₂ or R₃ is an optionally substituted phenyl group.

8. The agent according to claim 4, wherein X' is an alkyl group substituted with an optionally esterified carboxyl group.

9. The agent according to claim 4, wherein X' is an alkyl group substituted with an optionally substituted heterocyclic residue having a hydrogen atom that may be deprotonated.

10. The agent according to claim 9, wherein the heterocyclic residue is

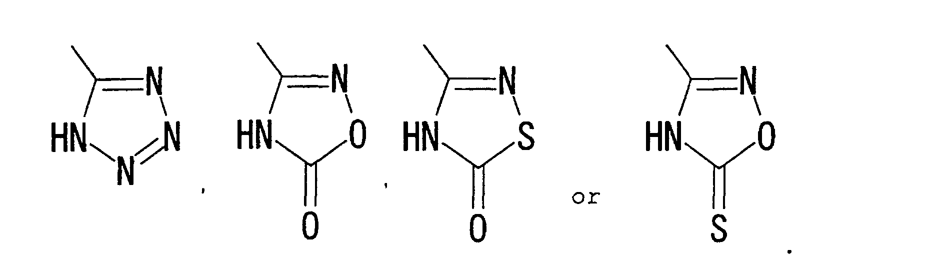

11. The agent according to claim 4, wherein X' is an alkyl group substituted with an optionally substituted carbamoyl group.

12. The agent according to claim 11, wherein the optionally substituted carbamoyl group is an optionally substituted cyclic aminocarbonyl group.

13. The agent according to claim 8, 9 or 11, wherein the alkyl group is a straight chain C₁₋₄ alkyl group.

14. The agent according to claim 4, wherein the heterocyclic ring represented by the ring A is

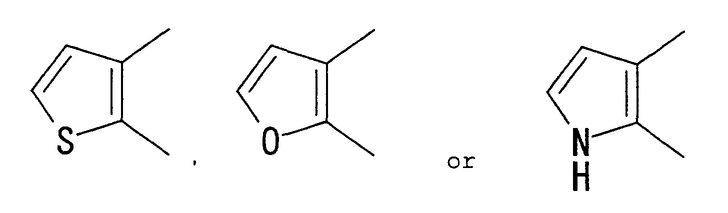

15. The agent according to claim 4, wherein the substituent of ring J' is an oxo or a thioxo.

16. The agent according to claim 4, wherein the fused ring consisting of the ring A and ring J' is

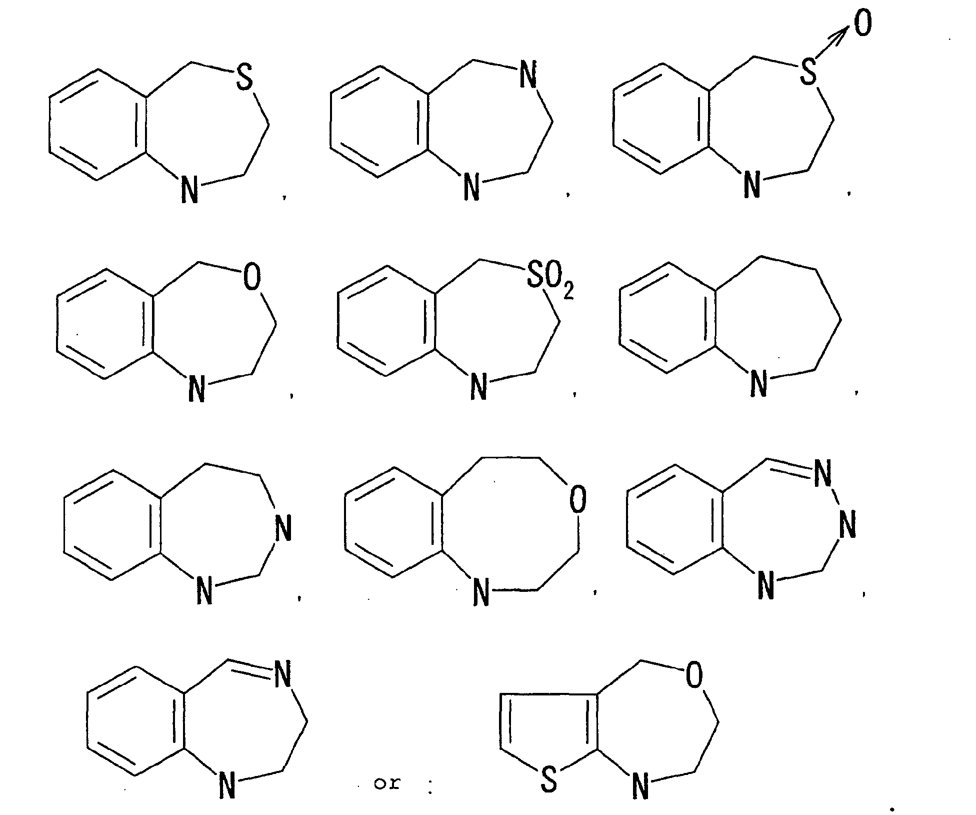

17. The agent according to claim 4, wherein R₂ and R₃ are each a hydrogen atom, an optionally substituted alkyl group, an optionally substituted phenyl group or an optionally substituted aromatic heterocyclic group.

18. The agent according to claim 1, comprising a compound represented by the formula: 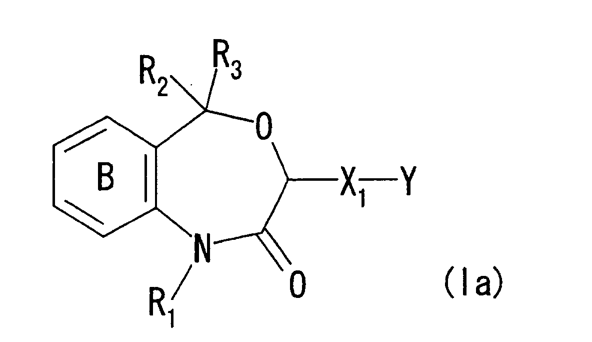 wherein R₁ is a hydrogen or an optionally substituted hydrocarbon group, R₂ and R₃ are the same or different and each is a hydrogen, an optionally substituted hydrocarbon group or an optionally substituted heterocyclic group, X₁ is a bond or divalent atomic chain, Y is an optionally esterified carboxyl group, an optionally substituted carbamoyl group, an optionally substituted hydroxy group, an optionally substituted amino group or an optionally substituted heterocyclic residue having a hydrogen atom which may be protonated and ring B is an optionally substituted benzene ring, or a salt thereof, or a prodrug thereof.

19. The agent according to claim 18, wherein R₁ is an optionally substituted aliphatic non-cyclic hydrocarbon group.

20. The agent according to claim 19, wherein the aliphatic non-cyclic hydrocarbon group is a branched alkyl group.

21. The agent according to claim 18, wherein R₂ or R₃ is an optionally substituted phenyl group.

22. The agent according to claim 18, wherein X₁ is a straight chain or a branched alkylene.

23. The agent according to claim 18, wherein X₁ is a straight chain C₁₋₄ alkylene.

24. The agent according to claim 18, wherein Y is an optionally esterified carboxyl group.

25. The agent according to claim 18, wherein Y is an optionally substituted carbamoyl group.

26. The agent according to claim 18, wherein Y is an optionally substituted heterocyclic residue having a hydrogen atom that may be deprotonated.

27. The agent according to claim 26, wherein the heterocyclic residue is

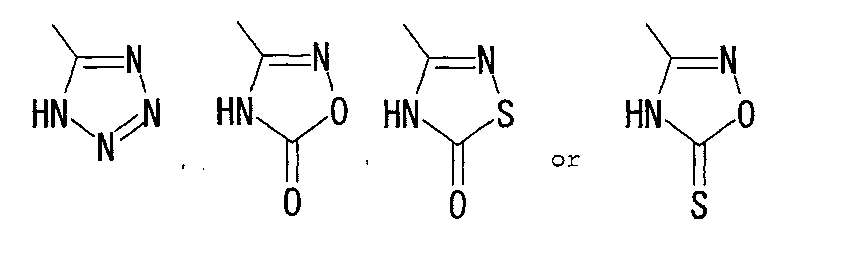

28. The agent according to claim 1, comprising a compound represented by the formula: 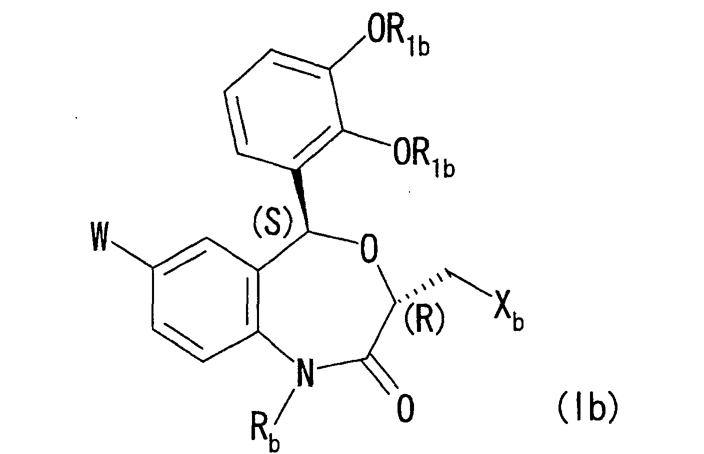 wherein R_{b} is a lower alkyl group optionally substituted with an optionally substituted hydroxy group, X_{b} is an optionally substituted carbamoyl group or an optionally substituted heterocyclic group having a hydrogen atom that may be deprotonated, R_{1b} is a lower alkyl group and W is a halogen atom or a salt thereof, or a prodrug thereof.

29. The agent according to claim 28, wherein R_{b} is a C₁₋₆ alkyl optionally having 1 to 3 substituents selected from a hydroxy group, an acetyloxy, a propionyloxy, a t-butoxycarbonyloxy, a palmitoyloxy, a dimethylaminoacetyloxy and a 2-aminopropionyloxy.

30. The agent according to claim 28, wherein R_{1b} is methyl.

31. The agent according to claim 28, wherein W is chlorine atom.

32. The agent according to claim 28, wherein X_{b} is a group represented by the formula: 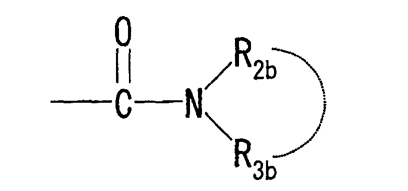 wherein R_{2b} and R_{3b} are each a hydrogen atom, an optionally substituted hydrocarbon group, an optionally substituted heterocyclic group or an acyl group, or R_{2b} and R_{3b} may form , together with the adjacent nitrogen atom, an optionally substituted 5- or 6-membered nitrogen-containing heterocyclic ring which may contain 1 to 3 heteroatoms selected from a nitrogen atom, a sulfur atom and an oxygen atom as the ring-constituting atoms.

33. The agent according to claim 28, wherein X_{b} is a group represented by the formula: 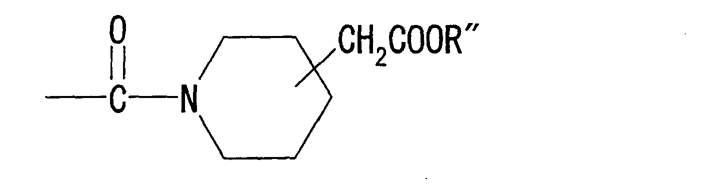 wherein R" is a hydrogen atom or a C₁₋₄ alkyl.

34. The agent according to claim 1, comprising a compound represented by the formula: 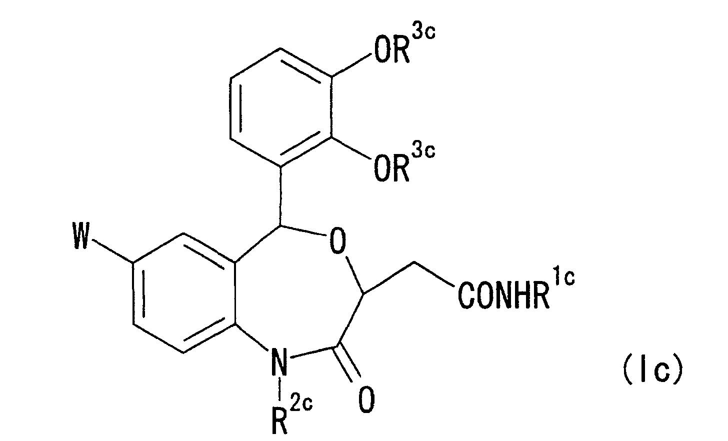 wherein R^{1c} is a 1-carboxyethyl group which may have substituent, a carboxy-C₃₋₆ straight chain alkyl group which may have substituent, a C₃₋₆ straight chain alkyl-sulfonyl group which may have substituent, a (carboxy-C₅₋₇ cycloalkyl)-C₁₋₃ alkyl group which may have substituent, or a group represented by the formula: -X¹-X²-Ar-X³-X⁴-COOH (wherein X¹ and X⁴ are each a bond or a C₁₋₄ alkylene group which may have substituent, X² and X³ are each a bond, -O-or -S- and Ar is a divalent aromatic ring group which may have substituent. Provided that when X¹ is a bond, X² is a bond, and when X⁴ is a bond, X³ is a bond) , R^{2c} is a C₃₋₆ alkyl group optionally substituted with an alkanoyloxy group and/or a hydroxy group, R^{3c} is a lower alkyl group and W is a halogen atom or a salt thereof, or a prodrug thereof.

35. The compound according to claim 34, wherein R^{1c} is a 3-carboxypropyl group; a 1-carboxyethyl group; or a C₃₋₆ straight chain alkyl-sulfonyl group, a (carboxy-C₅₋₇ cycloalkyl)-C₁₋₃ alkyl group, a (carboxyfuryl)-alkyl group, a carboxy-C₆₋₁₀ aryl group, a (carboxy-C₂₋₃ alkyl)-C₆₋₁₀ aryl group or a (carboxy-C₁₋₃ alkyl)-C₇₋₁₄ aralkyl group, each of which optionally has substituent.

36. The compound according to claim 34, wherein R^{1c} is a (carboxy-C₁₋₄ alkyl)-C₆₋₁₀ aryl group which may have substituent.

37. The compound according to claim 34, wherein R^{2c} is a C₃₋₆ alkyl group having an alkanoyloxy group and/or a hydroxy group.

38. The compound according to claim 34, wherein R^{2c} is a C₃₋₆ alkyl group which may have 1 to 3 substituents selected from a hydroxy group, an acetoxy, a propionyloxy, a t-butoxycarbonyloxy and a palmitoyloxy.

39. The compound according to claim 34, wherein R^{3c} is a methyl group.

40. The compound according to claim 34, wherein W is a chlorine atom.

41. The compound according to claim 34, wherein the 3-position has R-configuration and the 5-position has S-configuration.

42. The agent according to claim 1, comprising N-propanesulfonyl-[(3R,SS)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, (2R)-2-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminopropionic acid, 3-[3-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminophenyl]propionic acid, 4-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminobutanoic acid, trans-4-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl-1-cyclohexanecarboxylic acid, trans-4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl-1-cyclohexanecarboxylic acid, 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-fluorophenyl]propionic acid, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methylphenyl]propionic acid, 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-methylphenyl]propionic acid, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl]phenyl]propionic acid, 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl]phenyl]propionic acid, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]propionic acid, 2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid, 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazen-3-yl]acetyl]amino]-4-fluorophenyl]propionic acid, 3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid, N-methanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide, N-[2-(pyrrolidin-1-yl)ethyl]-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl] acetamide, N-[2-(pyrrolidin-1-yl)ethyl]-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-1-[3-acetoxy-2,2-dimethylpropyl)-7-chloro-5-(2,3-dimethoxyphenyl)-2-oxo-1,2,3,5-tetrahydro-4,1-benzoxazepin-3-yl]acetamide, N-methanesulfonyl-[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetamide, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid, N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester, N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester, (3R,5S)-7-chloro-5-(2,3-dimethoxyphenyl)-1-(3-hydroxy-2,2-dimethylpropyl)-1,2,3,5-tetrahydro-3-[1H (or 3H)-tetrazol-5-yl]methyl-4,1-benzoxazepin-2-one, (3R,5S)-7-chloro-5-(2,3-dimethoxyphenyl)-1-(3-hydroxy-2-hydroxymethyl-2-methylpropyl)-1,2,3,5-tetrahydro-3-[1H (or 3H)-tetrazol-5-yl]methyl-4,1-benzoxazepin-2-one, (3R,5S)-1-(3-acetoxy-2,2-dimethylpropyl-7-chloro-5-(2,3-dimethoxyphenyl)-1,2,3,5-tetrahydro-3-[lH (or 3H)-tetrazol-5-yl]methyl-4,1-benzoxazepin-2-one, (3R,5S)-1-(3-acetoxy-2-acetoxymethyl-2-methylpropyl)-7-chloro-5-(2,3-dimethoxyphenyl)-1,2,3,5-tetrahydro-3-[1H (or 3H)-tetrazol-5-yl]methyl-4,1-benzoxazepin-2-one, N-[2-(pyrrolidin-1-yl)ethyl]-[(3R,5S)-7-chloro-5-(2,3-dimethoxyphenyl)-1-neopentyl-2-oxo-1,2,3,5-tetrahydro-4,1-benzoxazepin-3-yl]acetamide, (3R,5S)-7-chloro-5-(2,3-dimethoxyphenyl)-1-neopentyl-2-oxo-1,2,3,5-tetrahydro-4,1-benzoxazepine-3-acetic acid, (3R,5S)-7-chloro-5-(2,4-dimethoxyphenyl)-1-neopentyl-2-oxo-1,2,3,5-tetrahydro-4,1-benzoxazepine-3-acetic acid, (3R,5S)-7-chloro-5-(4-ethoxy-2-methoxyphenyl)-1-neopentyl-2-oxo-1,2,3,5-tetrahydro-4,1-benzoxazepine-3-acetic acid, 4-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]butanoic acid, 5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]pentanoic acid, 5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]pentanoic acid,
or a pharmaceutically acceptable salt thereof, or a prodrug thereof.

43. An agent for preventing or treating familial hypercholesterolemia comprising a compound having squalene synthase inhibitory activity or a salt thereof, or a prodrug thereof.

44. A method for elevating high density lipoprotein-cholesterol in a mammal, comprising administering a compound having squalene synthase inhibitory activity or a salt thereof, or a prodrug thereof to said mammal in an effective amount.

45. A method for preventing or treating primary hypoalphalipoproteinemia in a mammal, comprising administering a compound having squalene synthase inhibitory activity or a salt thereof, or a prodrug thereof to said mammal in an effective amount.

46. A method for preventing or treating Tangier disease in a mammal, comprising administering a compound having squalene synthase inhibitory activity or a salt thereof, or a prodrug thereof to said mammal in an effective amount.

47. A method for preventing or treating familial hypercholesterolemia in a mammal, comprising administering a compound having squalene synthase inhibitory activity or a salt thereof, or a prodrug thereof to said mammal in an effective amount.

48. A method for preventing or treating hyperlipemia or arterial sclerosis, comprising elevating high density lipoprotein-cholesterol in a mammal by administering a compound having squalene synthase inhibitory activity or a salt thereof, or a prodrug thereof to said mammal in an effective amount.

49. Use of a compound having squalene synthase inhibitory activity or a salt thereof, or a prodrug thereof, for the production of a medicament for elevating high density lipoprotein-cholesterol.

50. Use of a compound having squalene synthase inhibitory activity or a salt thereof, or a prodrug thereof, for the production of a medicament for preventing or treating primary hypoalphalipoproteinemia.

51. Use of a compound having squalene synthase inhibitory activity or a salt thereof, or a prodrug thereof, for the production of a medicament for preventing or treating Tangier disease.

52. Use of a compound having squalene synthase inhibitory activity or a salt thereof, or a prodrug thereof, for the production of a medicament for preventing or treating familial hypercholesterolemia.
